(19) Europäisches Patentamt
European Patent Office
Office européen des brevets

(11) Publication number: 0 212 753 B1

(12) EUROPEAN PATENT SPECIFICATION

(45) Date of publication of the patent specification: 30.05.90

(21) Application number: 86201411.5

(22) Date of filing: 11.08.86

(51) Int. Cl.$^5$: C07C 257/22, C07C 251/72, C07D 207/06, C07D 231/18, C07D 241/08, C07D 249/14, C07D 333/38, A01N 37/52

(54) **Diphenyl ether Herbicides.**

(30) Priority: 20.08.85 GB 8520774

(43) Date of publication of application: 04.03.87 Bulletin 87/10

(45) Publication of the grant of the patent: 30.05.90 Bulletin 90/22

(84) Designated Contracting States: AT BE CH DE FR GB IT LI LU NL SE

(56) References cited:
EP-A- 0 046 445
DE-A- 2 113 997
GB-A- 1 185 950
GB-A- 2 049 695
GB-A- 2 152 501

The file contains technical information submitted after the application was filed and not included in this specification

(73) Proprietor: SHELL INTERNATIONALE RESEARCH MAATSCHAPPIJ B.V., Carel van Bylandtlaan 30, NL-2596 HR Den Haag(NL)

(72) Inventor: Munro, David, 40 Harvesters Way Maidstone, Kent(GB)
Inventor: Bit, Rino Antonio, 66 Northwood Drive Sittingbourne, Kent(GB)

(74) Representative: Bennett, David Arthur Horder et al, 4, York Road, London SE1 7NA(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 212 753 B1

## Description

This invention relates to certain diphenyl ether derivatives, the preparation of such compounds, herbicidal compositions containing them, and to their use in combating undesired plant growth.

Certain diphenyl ethers are known to be effective herbicides, for example UK Patent Specification No. 2 049 695A describes diphenyl ether ketone oximes and their use as herbicides. Also, European Patent Specification No. 46 445A1 describes methyleneamino substituted diphenyl ethers and Japanese Patent Specification No. 55-64 557 discloses certain hydrazino substituted diphenyl ethers.

It has now been found that useful and interesting herbicidal activity is present in diphenyl ethers bearing an amidrazone substituent grouping.

Accordingly, the present invention provides diphenyl ethers having the general formula I:

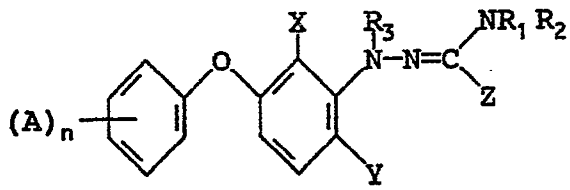

I

wherein: n is 0, 1, 2 or 3; each group A, which may be the same or different when n is greater than 1, independently represents a halogen, preferably chlorine, atom or a cyano, nitro, alkyl or haloalkyl, preferably trifluoromethyl, group;

X represents a hydrogen atom or, when n is 0 may alternatively represent a halogen, suitably chlorine, atom;

Y represents a halogen, suitably chlorine, atom or a cyano or, preferably, nitro group;

$R_1$ represents a hydrogen atom, an alkyl, cycloalkyl, alkenyl or alkynyl group, an alkyl group substituted by a halogen atom or a cyano or alkoxy group, or an optionally substituted aryl group;

$R_2$ represents a hydrogen atom, an alkyl, alkoxy, cycloalkyl, alkenyl or alkynyl group, an alkyl group substituted by a halogen atom or a cyano, alkoxy, alkylamino, acylamino, alkoxycarbonyl or acyl group, an optionally substituted aryl or heterocyclic group, or an amino group optionally substituted by an alkyl, aryl, alkanoyl, aroyl or alkoxycarbonyl group, or $R_1$ and $R_2$ together with the interjacent nitrogen atom jointly represent an azido group or a heterocyclic ring;

Z represents a hydrogen atom or a cyano, alkyl, alkylamino, carboxy, alkoxycarbonyl, or acyl group, or Z, $R_1$ and $R_2$, together with the interjacent carbon and nitrogen atoms represent a heterocyclic ring;

and $R_3$ represents a hydrogen atom or an alkyl, suitably methyl, group.

The term "acyl" is used herein to denote the radical derived from an organic acid by the removal of a hydroxyl group; the organic acid may be a carboxylic acid (including carbamic acid derivatives) or a sulphonic acid, examples of suitable acyl groups being alkylcarbonyl, phenylcarbonyl, carbamoyl optionally substituted by alkyl groups optionally bearing further substituents or by alkenyl, alkynyl or amino groups, alkylsulphonyl, and phenylsulphonyl, e.g. acetyl, (di)methylcarbamoyl, octylcarbamoyl, allylcarbamoyl, aminocarbamoyl, methoxyethylcarbamoyl.

When any of the foregoing substituents represents or contains an alkyl substituent group, this may be linear or branched and may contain up to 10, preferably up to 8, carbon atoms, suitable examples being methyl, ethyl, propyl, butyl and octyl. When they represent or contain a cycloalkyl substituent group this may contain from 3 to 10, preferably 5 to 8, carbon atoms, and is suitably cyclopropyl or cyclohexyl. When they contain a haloalkyl substituent group, this suitably contains up to 6, preferably up to 4, carbon atoms and the halogen atom is suitably fluorine or chlorine, trifluoromethyl being particularly preferred. When they are or contain an aryl substituent group, this is preferably a phenyl group. When they are or contain a heterocyclic group, this may, for example, be a pyrrole, pyrrolidine, pyridine, piperidine, oxazine, triazole, diazole, hydrodiazine, furan, thiophene or pyran ring. When any of the foregoing substituents are designated as being optionally substituted, the substituent groups which are optionally present may be any of those customarily employed in the development of pesticidal compounds, and/or the modification of such compounds to influence their structure/activity, persistence, penetration or other property. Specific examples of such substituents include halogen, especially chlorine, atoms and nitro, cyano, alkyl or haloalkyl, especially trifluoromethyl, groups.

One substituent A is desirably located para to the ether linkage, and the other substituent(s), when present, at the position(s) ortho to the ether linkage. Preferred compounds are those wherein n is 2 or 3; one group A is a haloalkyl, especially trifluoromethyl, group which is suitably located para to the ether linkage; the second group A is a halogen, especially chlorine, atom or a nitro or cyano group which is suitably located ortho to the ether linkage; and the third group A, when present, is a halogen, especially chlorine, atom.

Preferably X represents a hydrogen atom, Y represents a nitro group, and $R_3$ represents a hydrogen atom or an alkyl group of up to 6 carbon atoms, especially methyl.

$R_1$ is preferably a hydrogen atom or an optionally chlorinated alkyl group of up to 8 carbon atoms, especially methyl, ethyl, propyl or chloropropyl, or forms one of the combination groupings designated below.

$R_2$ is preferably a hydrogen atom, an alkyl group of up to 8 carbon atoms optionally bearing a halogen or alkoxy substituent, especially methyl, ethyl, propyl, butyl, octyl, trifluoropropyl, chloropropyl, chlorobutyl or methoxyethyl; a cyclopropyl or cyclohexyl group, optionally bearing an alkoxycarbonyl substituent; an alkenyl or alkynyl group of up to 8 carbon atoms, especially allyl or propynyl; a methyl or ethyl group bearing an alkoxycarbonyl, carbamoyl or alkylamino group; a phenyl group, an alkoxy, especially methoxy, group; a thiophene ring, optionally bearing an alkoxycarbonyl substituent; or an amino group optionally substituted by an alkyl, phenyl, acetyl, benzoyl or alkoxycarbonyl, especially methoxycarbonyl, group; or forms one of the combination groupings designated below.

Z is preferably a hydrogen atom or a methyl, cyano, carboxyl, alkylamino, especially methylamino, or phenylsulphonyl group; an alkoxycarbonyl or aralkoxycarbonyl group, especially methoxycarbonyl, ethoxycarbonyl, butoxycarbonyl, or benzyloxy carbonyl; an alkanoyl group, especially acetyl; or an N-substituted carbamoyl group of the formula $CONR_4R_5$, wherein $R_4$ represents a hydrogen atom or an alkyl group of up to 6 carbon atoms, especially methyl; and $R_5$ represents an alkyl group of up to 8 carbon atoms, suitably methyl, ethyl, propyl, or octyl, optionally bearing an alkoxy or alkylamino substituent; a cyclopropyl group; or an amino group optionally bearing an alkyl, alkenyl or alkynyl substituent, especially a methyl, allyl or propynyl substituent; or Z forms one of the combination groups designated below.

Alternatively, $R_1$ and $R_2$, or $R_1$, $R_2$ and Z, may be combined. Suitably $R_1$ and $R_2$, together with the interjacent nitrogen atom, represent an azido group or a nitrogen-containing heterocyclic ring selected from pyrrolidine, piperidine, hydro-oxazine or triazole; or $R_1$, $R_2$ and Z, together with the interjacent nitrogen and carbon atoms, represent a diazole or hydrodiazine ring.

The invention also provides a process for the preparation of hydrazino diphenyl ethers of formula I as defined above, which comprises reacting an amine of formula $HNR_1R_2$ with a hydrazonyl halide of general formula II:-

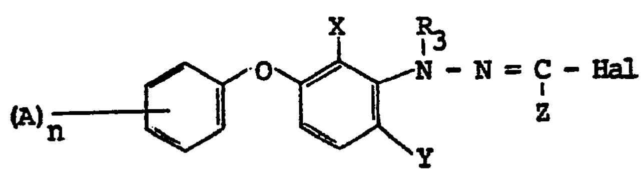

II

wherein Hal represents a halogen, preferably chlorine, atom; and the other substituents have the meanings defined above in relation to formula I. When $R_1$, $R_2$ and Z jointly with the interjacent nitrogen and carbon atoms, represent a cyclic group, this may be prepared by an analogous process in which the hydrazonyl halide of formula II is reacted with ethylene diamine or the appropriate derivative thereof. The reaction can be carried out at ambient temperature, but is usually expedited by the application of heat, conveniently by reacting under reflux.

The starting hydrazonyl halide of formula II may, when Z is not alkyl or hydrogen, be prepared by diazotization of an aniline derivative of general formula III:-

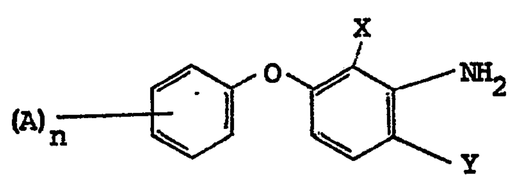

III

wherein n, A, X and Y are as defined in relation to formula I above, and coupling with a haloacyl compound of general formula IV:

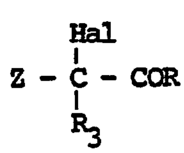

IV

wherein Hal represents a halogen, preferably chlorine, atom, R represents an alkyl, conveniently methyl, group, and Z and $R_3$ are as defined above in relation to formula I (except that Z is not alkyl or hydrogen).

The diazotization and coupling of the compound of formula III are carried out under the conditions well-established for such types of reaction, namely using an alkali metal nitrite and mineral acid at a temperature between 0°C and 10°C for the former, and reaction at ambient temperature for the latter.

The starting hydrazonyl halide of formula II may, when Z is an alkyl group or a hydrogen atom, be prepared by reacting a hydrazine of formula $R_3NH\text{-}NH_2$ with a nitro compound of formula V

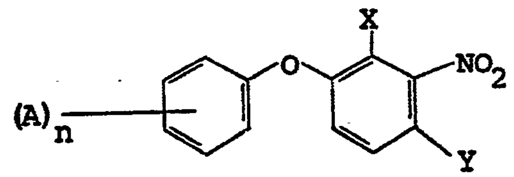

V

acylating the resulting phenyl hydrazine by reaction with an acid or its anhydride or an acyl halide of formula ZCOOH or ZCOHal, wherein Z is alkyl or hydrogen and Hal represents a halogen, preferably chlorine, atom, followed by reaction with phosphorus pentachloride. The reaction of the nitro and hydrazine compounds, and the subsequent acylation, are each preferably carried out in an inert organic solvent, such as dioxan or toluene, and the acylation is conveniently expedited by the presence of a hydrogen halide acceptor, suitably an organic base such as triethylamine. The reaction with phosphorus pentachloride is preferably carried out by warming, suitably to between 60-80°C, in the absence of a solvent.

When the desired final product is a compound of formula I in which Z represents an alkylamino group, this may be obtained by amination of a hydrazonyl halide of formula II wherein Z represents sulphonyl. That compound contains two leaving groups, viz the halogen atom and the sulphonyl group and reaction with the amine $HNR_1R_2$ thus results in the displacement of both groups with $-NR_1R_2$, thereby yielding the product wherein Z is alkylamino.

When the desired final product is one in which Z represents a carbamoyl derivative, this may be obtained by use of the appropriate haloacyl compound wherein Z is carbamoyl, or alternatively, if convenient, via the synthesis of a compound of formula II wherein Z is alkoxycarbonyl, e.g. methoxycarbonyl, reaction of that hydrazonyl halide with an amine to displace the halogen atom by $-NR_1R_2$, followed by further reaction of the product with the same or a different amine to displace the alkoxy moiety of the alkoxycarbonyl substituent at Z. Depending on the steric and electronic properties of the base $HNR_1R_2$, the alkoxy displacement may take place concurrently with the displacement of the halogen atom.

The compounds of general formula I have been found to show interesting activity as herbicides. Accordingly, the invention further provides a herbicidal composition comprising a compound of formula I as defined above in association with at least one carrier, and a method of making such composition which comprises bringing a compound of formula I into association with at least one carrier.

The invention also provides the use of such compound or composition according to the invention as a herbicide. Further, in accordance with the invention there is provided a method of combating undesired plant growth at a locus by treating the locus with a compound or composition according to the invention. Application to the locus may be pre-emergence or post-emergence. The dosage of active ingredient used may, for example, be from 0.05 to 4 kg/ha.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides

containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, or sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½-10% w of active ingredient. Granules are usually prepared to have a size between 10 and 100 BS mesh (1.676 - 0.152 mm), and may be manufactured by agglomeration or impregnation techniques. Generally, granules will contain ½-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties.

The invention is illustrated in the following Examples.

## Example 1

A) (2'-chloro-4'-trifluoromethylphenoxy)-3,4-dinitrobenzene (100g) was dissolved in dioxane (150 ml), and ammonia bubbled through the solution for 24 hours. The solvent was removed in vacuo and chloroform/water (1:1;400ml) added to the residue. The organic layer was separated, dried, evaporated, and recrystallised to yield yellow crystals (m.pt. 82°C) of 5-(2'-chloro-4'-trifluoromethylphenoxy)-2-nitro aniline.

B) The phenoxy aniline product of A) (6.8g) was dissolved in a minimum quantity of acetic acid and conc. hydrochloric acid (6.5ml) and ice/water (10ml) were added. This suspension was then treated with sodium nitrite (1.5g) in water (3ml) at 8°C for 1½ hours.

A solution of methyl-2-chloroacetoacetate (3g) and sodium acetate (0.4g) in water (10ml) and ethanol (12ml) was added at 6°C to the product of the foregoing diazotization, the pH being maintained at about 4.5 by the dropwise addition of 33%w/v aqueous sodium hydroxide. After standing for 2 hours, ether/water (1:1,400ml) was added, and the organic layer separated, washed, dried, and chromatographically separated to yield a yellow oil, which on recrystallisation gave yellow crystals, m.pt. 150°C of the compound:-

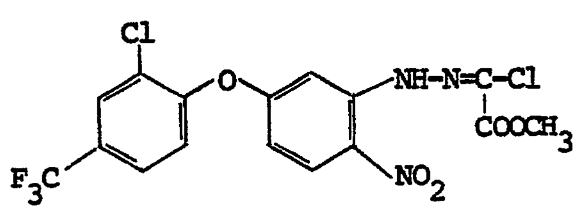

C) The hydrazino acid product of B) (3g) was dissolved in chloroform (40ml) and methylamine bubbled through at ambient temperature. Chromatographic separation of the reaction mixture, followed by recrystallisation, gave a reddish-brown solid, m.pt. 183°C of the desired product:

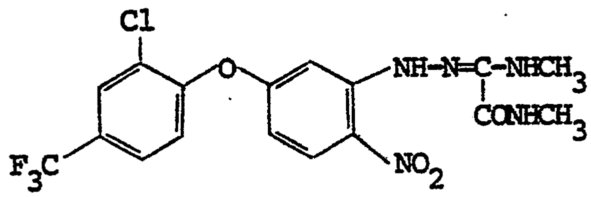

| | | | | |
|---|---|---|---|---|
| Analysis | Calc. for $C_{17}H_{15}N_5ClF_3O_4$: | C 45.8; | H 3.4; | N 15.7% |
| | Found: | C 45.7; | H 3.4; | N 15.7% |

Example 2

The hydrazino acid product of Example 1(B) (2g) was dissolved in chloroform (20ml) and methoxylamine hydrochloride (0.8g) added followed by triethylamine (2g). The reaction mixture was stirred at ambient temperature for 2 hrs and then refluxed for 3 hrs. Chromatographic separation of the reaction mixture, followed by recrystallisation, gave orange crystals, m.pt. 194°C, of the desired product:

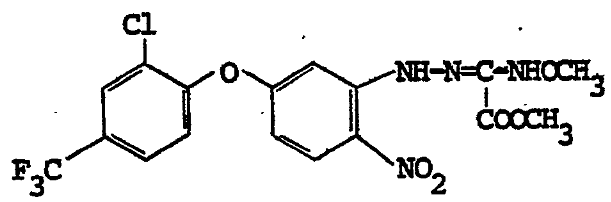

| | | | | |
|---|---|---|---|---|
| Analysis | Calc. for $C_{17}H_{14}N_4ClF_3O_6$: | C 44.1; | H 3.0; | N 12.2% |
| | Found: | C 44.2; | H 3.0; | N 12.6% |

Example 3

The hydrazino acid product of Example 1(B) (2g) was dissolved in chloroform (20ml) and glycine methyl ester hydrochloride (0.75g) added, followed by excess triethylamine (2g). The reaction mixture was stirred at ambient temperature for 2 hrs followed by reflux for 1 hr. Chloroform/water (1:1; 100ml) was added, and the organic layer was separated and dried. Chromatographic purification, followed by recrystallisation, yielded orange crystals, m.pt. 170°C of the desired product:

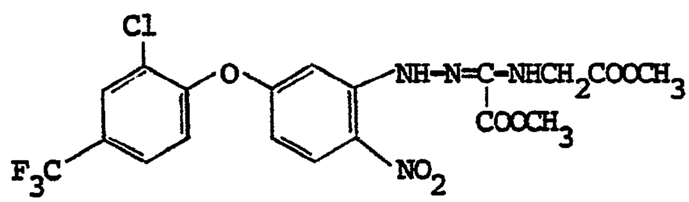

| | | | | |
|---|---|---|---|---|
| Analysis | Calc. for $C_{19}H_{16}N_4ClF_3O_7$: | C 45.2; | H 3.2; | N 11.1% |
| | Found: | C 45.2; | H 3.1; | N 10.9% |

Example 4

Dimethylamine (20ml) was added dropwise to a stirred solution of the hydrazino acid product of 1(B) (2.8g) in chloroform (50ml) at 5°C. The reaction mixture was stirred at 5°C for 30 mins, after which the solvent was removed. The residual oil was chromatographically separated to yield, as a red solid, m.pt. 103-104°C, the desired product of formula:-

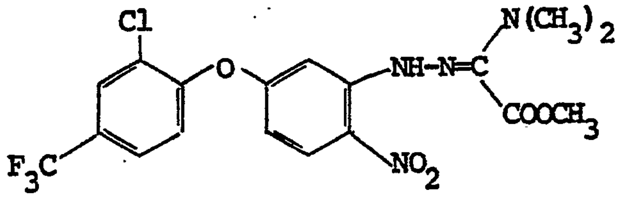

| | | | | |
|---|---|---|---|---|
| Analysis | Calc. for $C_{18}H_{16}ClF_3N_4O_5$: | C 46.4; | H 3.5; | N 12.2% |
| | Found: | C 47.0; | H 3.5; | N 12.0% |

Example 5

Following a procedure similar to that described in Example 4, but replacing the dimethylamine with ethylenediamine, the following compound was obtained as a red solid, m.pt. 210-215°C (decomp.)

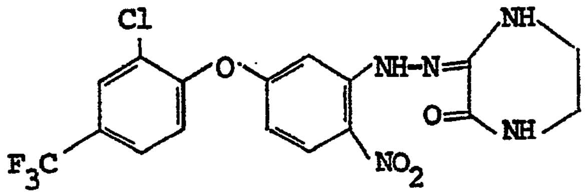

| | | | | |
|---|---|---|---|---|
| Analysis | Calc. for $C_{17}H_{13}ClF_3N_5O_4$: | C 46.0; | H 2.9; | N 15.8% |
| | Found: | C 46.2; | H 2.9; | N 15.3% |

Examples 6-69

Following procedures similar to those described in the foregoing Examples, additional examples of compounds of the invention were prepared, whose melting points and chemical analyses are given in Table 1 below. In that Table, the compounds are identified by reference to the identity of the substituents in the following formula:-

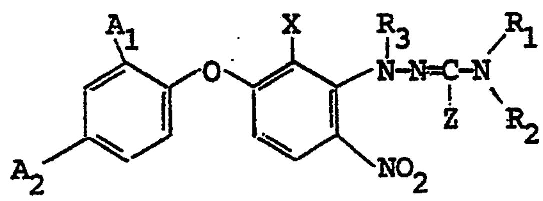

(In Ex. 54, $A_1$ and $A_2$ are both H and X is Cl. In all other Examples, $A_1$ is Cl; $A_2$ is $CF_3$; and X is H). In the tables below 'R' and 'F' respectively denote 'required' and 'found'.

Table I

| Example No. | $R_1$ | $R_2$ | Z | $R_3$ | M.Pt. °C | Analysis %C | %H | %N | |
|---|---|---|---|---|---|---|---|---|---|
| 6 | H | H | $COOCH_3$ | H | 183 | 44.4 | 2.8 | 12.9 | R |
| | | | | | | 44.5 | 2.8 | 12.5 | F |
| 7 | H | $C_2H_5$ | $CONHC_2H_5$ | H | 160 | 48.2 | 4.0 | 14.8 | R |
| | | | | | | 48.0 | 3.7 | 14.9 | F |
| 8 | H | $C_2H_5$ (inverse Isomer) | $COOCH_3$ | H | 188 | 46.9 | 3.4 | 12.2 | R |
| | | | | | | 47.0 | 3.5 | 12.2 | F |
| 9 | H | Phenyl | $COOCH_3$ | H | 190 | 51.9 | 3.2 | 11.0 | R |
| | | | | | | 51.9 | 3.2 | 10.9 | F |
| 10 | 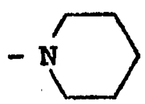 | | $COOCH_3$ | H | 159 | 50.4 | 3.8 | 11.2 | R |
| | | | | | | 50.7 | 3.9 | 11.0 | F |
| 11 | 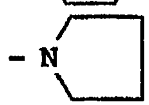 | | $COOCH_3$ | H | 92 | 49.4 | 3.5 | 11.2 | R |
| | | | | | | 49.5 | 3.1 | 10.8 | F |

Table I (continued)

| Example No. | $R_1$ | $R_2$ | Z | $R_3$ | M.Pt. °C | Analysis %C | %H | %N | |
|---|---|---|---|---|---|---|---|---|---|
| 12 | 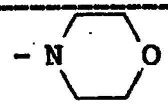 | | $COOCH_3$ | H | 166 | 47.9 | 3.4 | 11.2 | R |
| | | | | | | 47.7 | 3.4 | 11.3 | F |
| 13 | $CH_3$ | $OCH_3$ | $COOCH_3$ | H | 180 | 45.2 | 3.3 | 11.8 | R |
| | | | | | | 45.8 | 3.3 | 12.4 | F |
| 14 | H | $(CH_2)_2OCH_3$ | $CONH(CH_2)_2OCH_3$ | H | 106 | 47.2 | 4.3 | 13.1 | R |
| | | | | | | 46.1 | 4.2 | 12.4 | F |
| 15 | $CH_3$ | $NH_2$ | $COOCH_3$ | H | 151 | 44.2 | 3.3 | 18.2 | R |
| | | | | | | 43.6 | 3.4 | 18.2 | F |
| 16 | H | $NH_2$ | $CONHNH_2$ | H | 169 | 40.2 | 2.4 | 21.9 | R |
| | | | | | | 40.4 | 2.6 | 22.2 | F |
| 17 | $CH_3$ | $NHCH_3$ | $COOCH_3$ | H | 120 | 45.4 | 3.6 | 14.7 | R |
| | | | | | | 45.6 | 3.5 | 14.4 | F |
| 18 | H | 2-$CH_3COO$ thiophene | $COOCH_3$ | H | 214 | 46.1 | 2.8 | 9.8 | R |
| | | | | | | 46.7 | 2.7 | 9.6 | F |
| 19 | $CH_3$ | $CH_3$ | $CONHCH_3$ | H | 175 | 47.0 | 3.7 | 15.2 | R |
| | | | | | | 47.0 | 3.3 | 15.0 | F |

Table I (continued)

| Example No. | $R_1$ | $R_2$ | Z | $R_3$ | M.Pt. °C | Analysis %C | %H | %N | |
|---|---|---|---|---|---|---|---|---|---|
| 20 | H | NHPhenyl | $COOCH_3$ | H | 161 | 50.4 | 3.2 | 13.4 | R |
| | | | | | | 50.6 | 3.2 | 13.1 | F |
| 21 | H | $N(CH_3)_2$ | $CONHN(CH_3)_2$ | H | 117 | 45.4 | 3.6 | 14.7 | R |
| | | | | | | 45.8 | 3.4 | 14.3 | F |
| 22 | H | $CH_3$ | $COCH_3$ | H | 110 | 47.4 | 3.2 | 13.0 | R |
| | | | | | | 47.4 | 3.0 | 12.8 | F |
| 23 | $CH_3$ | $CH_3$ | $COCH_3$ | H | 114 | 48.6 | 3.6 | 12.6 | R |
| | | | | | | 48.6 | 3.7 | 12.6 | F |
| 24 | H | $NHCOOCH_3$ | $COOCH_3$ | H | 178 | 42.7 | 3.0 | 13.8 | R |
| | | | | | | 42.8 | 2.9 | 13.7 | F |
| 25 | H | Cyclohexyl | $COOCH_3$ | H | 114 | 51.3 | 4.3 | 10.9 | R |
| | | | | | | 51.0 | 4.3 | 10.8 | F |
| 26 | H | $C_4H_9^t$ | $COOCH_3$ | H | 140 | 49.1 | 4.1 | 11.5 | R |
| | | | | | | 48.9 | 4.0 | 11.4 | F |
| 27 | H | $(CH_2)_7CH_3$ | $CONH(CH_2)_7CH_3$ | H | 97 | 58.0 | 6.7 | 10.9 | R |
| | | | | | | 58.0 | 6.8 | 10.5 | F |

Table I (continued)

| Example No. | $R_1$ | $R_2$ | Z | $R_3$ | M.Pt.°C | Analysis %C | %H | %N | |
|---|---|---|---|---|---|---|---|---|---|
| 28 | H | $CH_2CONH_2$ | $COOCH_3$ | H | 205 | 44.1 | 3.1 | 14.3 | R |
| | | | | | | 44.1 | 3.0 | 13.6 | F |
| 29 | H | $C_3H_7^n$ | $CONHC_3H_7^n$ | H | 134 | 52.1 | 5.1 | 13.2 | R |
| | | | | | | 51.8 | 5.3 | 12.7 | F |
| 30 | H | $C_2H_5Cl$ | $COOCH_3$ | H | 102 | 43.6 | 3.0 | 11.4 | R |
| | | | | | | 43.7 | 2.9 | 11.4 | F |
| 31 | H | $C_3H_7Cl$ | $COOCH_3$ | H | 143 | 44.8 | 3.3 | 11.0 | R |
| | | | | | | 45.2 | 3.4 | 11.3 | F |
| 32 | H | $CH_2CF_3$ | $COOCH_3$ | H | 109 | 42.0 | 2.5 | 10.9 | R |
| | | | | | | 41.9 | 2.8 | 11.1 | F |
| 33 | H | $CH_2CH{=}CH_2$ | $CONHCH_2CH{=}CH_2$ | H | 161 | 50.1 | 3.8 | 14.1 | R |
| | | | | | | 49.7 | 3.7 | 13.6 | F |
| 34 | H | $CH_2C{\equiv}CH$ | $CONHCH_2C{\equiv}CH$ | H | 165 | 51.1 | 3.0 | 14.2 | R |
| | | | | | | 49.9 | 3.0 | 14.3 | F |
| 35 | H | $NHCOCH_3$ | $COOCH_3$ | H | 216 | 44.1 | 3.1 | 14.3 | R |
| | | | | | | 44.1 | 3.2 | 14.3 | F |

Table I (continued)

| Example No. | $R_1$ | $R_2$ | Z | $R_3$ | M.Pt.°C | Analysis %C | %H | %N | |
|---|---|---|---|---|---|---|---|---|---|
| 36 | H | NHCOPhenyl | $COOCH_3$ | H | 186 | 50.0 | 3.1 | 12.7 | R |
| | | | | | | 50.2 | 3.2 | 12.6 | F |
| 37 | H | $(CH_2)_2N(CH_3)_2$ | $CONH(CH_2)_2N(CH_3)_2$ | H | 112 | 49.3 | 5.2 | 17.5 | R |
| | | | | | | 49.1 | 5.2 | 17.3 | F |
| 38 | | 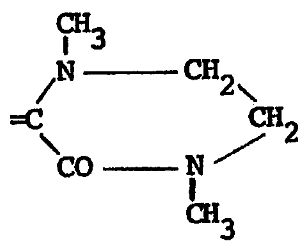 | | H | 198 | 48.4 | 3.6 | 14.8 | R |
| | | | | | | 48.0 | 3.8 | 15.0 | F |
| 39 | 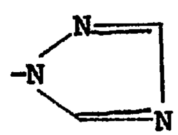 | | $COOCH_3$ | H | 191 | 44.6 | 2.5 | 17.3 | R |
| | | | | | | 45.3 | 2.5 | 16.5 | F |
| 40 | $=\overset{+}{N}=\overset{-}{N}$ | | $COOCH_3$ | H | 156 | 41.9 | 2.2 | 18.3 | R |
| | | | | | | 42.2 | 2.3 | 18.0 | F |
| 41 | $C_2H_5$ | $C_2H_5$ | $COOCH_3$ | H | 94 | 49.1 | 4.1 | 11.5 | R |
| | | | | | | 49.3 | 4.1 | 11.5 | F |

Table I (continued)

| Example No. | $R_1$ | $R_2$ | Z | $R_3$ | M.Pt.°C | Analysis %C | %H | %N | |
|---|---|---|---|---|---|---|---|---|---|
| 42 | H | 1-$COOC_2H_5$ Cyclopropyl | $COOCH_3$ | H | 160 | 48.5 | 3.7 | 10.3 | R |
| | | | | | | 48.5 | 3.6 | 10.3 | F |
| 43 | 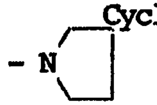 | | $CONHCH_3$ | H | 221 | 44.4 | 3.9 | 14.4 | R |
| | | | | | | 44.0 | 4.1 | 14.2 | F |
| 44 | $CH_3$ | $CH_3$ (Isomer 1) | $CONHC_2H_5$ | H | 190 | 48.1 | 4.0 | 14.8 | R |
| | | | | | | 48.1 | 4.1 | 15.1 | F |
| 45 | $CH_3$ | $CH_3$ (Isomer 2) | $CONHC_2H_5$ | H | 189 | 48.1 | 4.0 | 14.8 | R |
| | | | | | | 47.5 | 3.9 | 14.7 | F |
| 46 | $CH_3$ | $CH_3$ | $CONH(CH_2)_7CH_3$ | H | 100 | 53.8 | 5.6 | 12.6 | R |
| | | | | | | 53.5 | 5.8 | 12.7 | F |
| 47 | H | $CH_3$ | $CON(CH_3)_2$ | H | 175 | 47.0 | 3.7 | 15.2 | R |
| | | | | | | 47.2 | 3.8 | 15.2 | F |
| 48 | $CH_3$ | $CH_3$ | $CON(CH_3)_2$ | H | 98 | 48.2 | 4.0 | 14.8 | R |
| | | | | | | 48.3 | 4.0 | 14.8 | F |
| 49 | H | Cyclopropyl | $COOCH_3$ | H | 161 | 48.3 | 3.4 | 11.9 | R |
| | | | | | | 47.8 | 3.3 | 11.8 | F |

Table I (continued)

| Example No. | $R_1$ | $R_2$ | Z | $R_3$ | M.Pt.°C | Analysis %C | %H | %N | |
|---|---|---|---|---|---|---|---|---|---|
| 50 | H | Cyclopropyl | CONHCyclopropyl | H | 198 | 50.7 | 3.8 | 14.1 | R |
| | | | | | | 51.0 | 4.2 | 13.9 | F |
| 51 | H | $CH_3$ | CN | H | 184 | 46.4 | 2.7 | 16.9 | R |
| | | | | | | 46.4 | 2.7 | 16.8 | F |
| 52 | $CH_3$ | $CH_3$ | CN | H | oil | 47.7 | 3.1 | 16.4 | R |
| | | | | | | 47.7 | 3.1 | 16.4 | F |
| 53 | $CH_3$ | $CH_3$ | $COOCH_3$ | H | 105 | 46.9 | 3.5 | 12.2 | R |
| | | (Isomer 2.vs Ex.5) | | | | 46.9 | 3.3 | 12.1 | F |
| 54 | H | $CH_3$ | $CONHCH_3$ | H | 158 | 50.8 | 4.2 | 18.5 | R |
| | | | | | | 50.8 | 4.2 | 18.5 | F |
| 55 | | 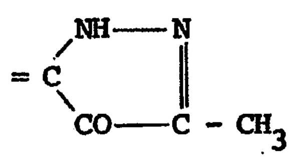 | | H | 200 | 46.2 | 2.5 | 15.8 | R |
| | | | | | | 46.2 | 2.5 | 15.8 | F |

## Table I (continued)

| Example No. | $R_1$ | $R_2$ | Z | $R_3$ | M.Pt. °C | Analysis %C | %H | %N | |
|---|---|---|---|---|---|---|---|---|---|
| 56 | H | $C_2H_5Cl$ | $CONHCH_3$ | H | 163 | 43.7 | 3.2 | 14.2 | R |
| | | | | | | 45.2 | 3.9 | 14.6 | F |
| 57 | $C_2H_5Cl$ | $C_2H_5Cl$ | $COOCH_3$ | H | oil | 43.2 | 3.6 | 9.9 | R |
| | | | | | | 44.1 | 3.7 | 9.8 | F |
| 58 | $C_2H_5Cl$ | $C_2H_5Cl$ | $CONHCH_3$ | H | 149 | 43.1 | 3.4 | 12.6 | R |
| | | | | | | 43.4 | 3.4 | 12.6 | F |
| 59 | $CH_3$ | $CH_2C{\equiv}CH$ | $COOCH_3$ | H | 128 | 49.6 | 3.3 | 11.5 | R |
| | | | | | | 49.7 | 3.3 | 11.4 | F |
| 60 | $CH_3$ | $CH_2C{\equiv}CH$ | $CONHCH_3$ | H | 110 | 49.6 | 3.5 | 14.5 | R |
| | | | | | | 49.6 | 3.6 | 14.5 | F |
| 61 | H | $CH_2C{\equiv}CH$ | $COOCH_3$ | H | 186 | 48.5 | 3.0 | 11.9 | R |
| | | | | | | 48.7 | 3.0 | 11.9 | F |
| 62 | $CH_3$ | $CH_3$ | $COOC_4H_9^t$ | H | oil | 50.1 | 4.4 | 11.1 | R |
| | | | | | | 49.6 | 4.3 | 10.5 | F |
| 63 | H | $CH_3$ | $COOC_4H_9^t$ | H | 164 | 49.1 | 4.1 | 11.5 | R |
| | | | | | | 49.4 | 4.2 | 11.7 | F |

Table I (continued)

| Example No. | $R_1$ | $R_2$ | Z | $R_3$ | M.Pt.°C | Analysis %C | %H | %N | |
|---|---|---|---|---|---|---|---|---|---|
| 64 | H | $CH_2C{\equiv}CH$ | $CONHCH_3$ | H | 196 | 48.6 | 3.2 | 14.9 | R |
| | | | | | | 47.9 | 3.4 | 14.7 | F |
| 65 | $CH_3$ | $CH_3$ | $COOCH_2Ph$ | H | oil | 53.7 | 3.7 | 10.4 | R |
| | | | | | | 53.8 | 3.7 | 9.9 | F |
| 66 | $CH_3$ | $CH_3$ | COOH | H | 166 | 45.7 | 3.1 | 12.5 | R |
| | | | | | | 45.0 | 3.2 | 12.5 | F |
| 67 | $CH_3$ | $CH_3$ | H | H | 163 | 47.7 | 3.5 | 13.9 | R |
| | | | | | | 48.2 | 3.7 | 13.7 | F |
| 68 | $CH_3$ | $CH_3$ | H | $CH_3$ | oil | 49.9 | 3.8 | 13.4 | R |
| | | | | | | 50.1 | 4.2 | 13.4 | F |

Example 69

Following a procedure similar to that described in Example 1, sections A and B, but substituting 1-chloro-1-phenyl-sulphonyl-propan-2-one for the methyl-2-chloroacetoacetate, there was obtained a hydrazino sulphonyl compound of formula:-

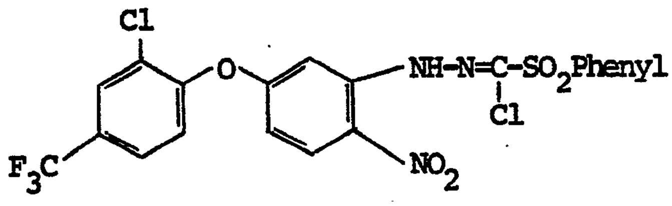

This hydrazino sulphonyl product (0.75g) was dissolved in chloroform (50ml) and methylamine bubbled through at 0°C. After 30 mins, the solvent was removed in vacuo, and the residual red oil chromatographically separated to yield red crystals, m.pt. 218-219 of the product:

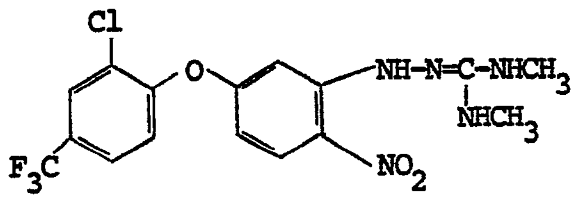

| | | | | |
|---|---|---|---|---|
| Analysis | Calc. for $C_{16}H_{15}ClF_3N_5O_3$: | C 46.0; | H 3.6; | N 16.8% |
| | Found: | C 46.2; | H 3.7; | N 16.5% |

Example 70

Following a procedure similar to that of Example 69, but replacing the methylamine with dimethylamine, there was obtained, as red crystals m.pt. 123-125°C, the compound

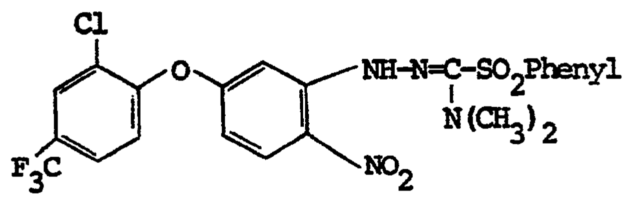

| | | | | |
|---|---|---|---|---|
| Analysis | Calc. for $C_{22}H_{18}ClF_3N_4O_5S$: | C 48.7; | H 3.3; | N 10.3% |
| | Found: | C 49.0; | H 3.3; | N 10.4% |

Example 71

A) (2'-chloro-4'-trifluoromethylphenoxy)-3,4-dinitro benzene (5g) was dissolved in dioxan (50ml) and methylhydrazine (0.8g) added dropwise with stirring under nitrogen, with immediate reaction. The reaction mixture was chromatographically separated and recrystallised to yield the 3-(alpha methyl)hydrazine derivative, m.pt 100°C.

B) The phenyl hydrazine product of A) (8.2g) was taken up in glacial acetic acid (100ml) and acetic anhydride (3g) was added with stirring under nitrogen at ambient temperature. The reaction mixture was then heated to 90°C with stirring for 3 hrs. before pouring into water (800ml) and extracting with ethyl acetate (400ml). The organic layer was separated, washed, dried, chromatographically purified, and the product recrystallised to yield yellow crystals, m.pt. 153°C, of:

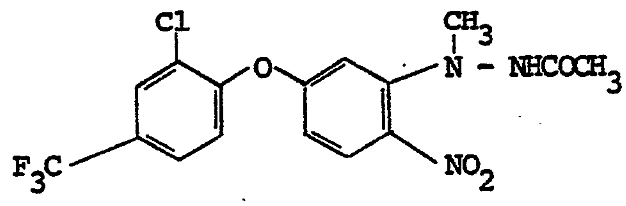

C) The product of B) (3g) and phosphorus pentachloride (2.1g) were ground in a pestle/mortar, stirred under nitrogen, and then heated at 70°C for 1½ hrs. Benzene (100ml) was added, the mixture allowed to cool and excess dimethylamine added. The solvent was evaporated off until the volume had been halved, chloroform/water (1:1, 600ml) added, and the organic layer separated, washed and dried. Chromatographic purification yielded a red oil of the desired product.

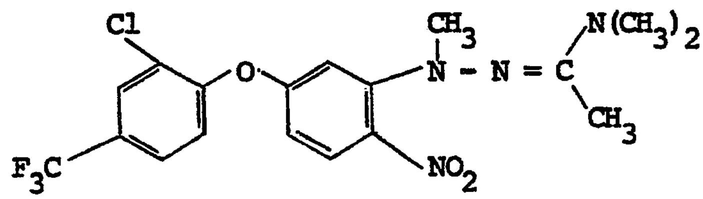

| | | | | |
|---|---|---|---|---|
| Analysis | Calc. for $C_{18}H_{18}N_4ClF_3O_3$: | C 50.1; | H 4.2; | N 13.0% |
| | Found: | C 49.5; | H 4.3; | N 12.8% |

Examples 72-75

Following procedures similar to those described in Example 71, additional examples of compounds of the invention were prepared, whose melting points and chemical analyses are given in Table II below. In that Table, the compounds are identified by reference to the identity of the substituents in the following formula:-

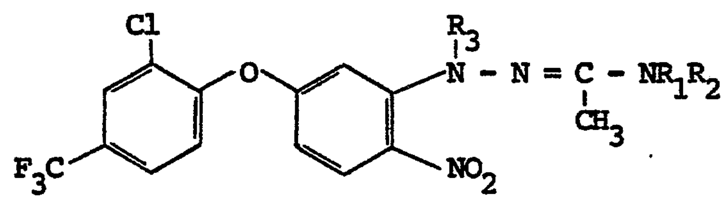

## Table II

| Example No. | $R_1$ | $R_2$ | Z | $R_3$ | M.Pt.°C | Analysis %C | %H | %N | |
|---|---|---|---|---|---|---|---|---|---|
| 72 | $C_3H_7$ | $C_3H_7$ | $CH_3$ | H | oil | 53.3 | 5.1 | 11.8 | R |
| | | | | | | 53.3 | 4.9 | 11.8 | F |
| 73 | H | $CH_3$ | $CH_3$ | H | 166 | 47.7 | 3.5 | 13.9 | R |
| | | | | | | 46.2 | 3.3 | 13.6 | F |
| 74 | $CH_3$ | $CH_3$ | $CH_3$ | H | 112 | 48.9 | 3.8 | 13.4 | R |
| | | | | | | 48.8 | 3.9 | 13.4 | F |
| 75 | H | $CH_3$ | $CH_3$ | $CH_3$ | 105 | 49.0 | 3.9 | 13.5 | R |
| | | | | | | 49.0 | 4.0 | 13.5 | F |

Example 76 Herbicidal Activity

To evaluate their herbicidal activity, compounds according to the invention were tested using as a representative range of plants: maize, Zea mays (Mz); rice, Oryza sativa (R); barnyard grass, Echinochloa crusgalli (BG); oat, Avena sativa (O); linseed, Linum usitatissisum (L); mustard, Sinapsis alba (M); sugar beet, Beta vulgaris(SB) and soya bean, Glycine max (S).

The tests fall into two categories, pre-emergence and post-emergence. The pre-emergence tests involved spraying a liquid formulation of the compound onto the soil in which the seeds of the plant species mentioned above had recently been sown. The post-emergence tests involved two types of test, viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horticultural loam.

The formulations used in the tests were prepared from solutions of the test compounds in acetone containing 0.4% by weight of an alkylphenol/ethylene oxide condensate available under the trade mark TRITON X-155. These acetone solutions were diluted with water and the resulting formulations applied at dosage levels corresponding to 5 kg or 1 kg of active material per hectare in a volume equivalent to 600 litres per hectare in the soil spray and foliar spray tests, and at a dosage level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually twelve days after spraying the foliage and the soil, and thirteen days after drenching the soil and were recorded on a 0-9 scale. A rating 0 indicates growth as untreated control, a rating 9 indicates death. An increase of 1 unit on the linear scale approximates to a 10% increase in the level of effect.

The results of the tests are set out in Table III below.

TABLE III

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 1 | | | | | | | | | 5 | 5 | 6 | 8 | 7 | 8 | 6 | 6 | 4 | 7 | 7 | 9 | 8 | 7 | 6 | 8 | 7 |
| | | | | | | | | | 1 | 2 | 2 | 6 | 5 | 5 | 4 | 4 | 4 | 6 | 5 | 9 | 7 | 7 | 4 | 7 | 7 |
| 2 | | | | | | | 3 | | 5 | 4 | 2 | 7 | 6 | 8 | 8 | 7 | 3 | 8 | 6 | 9 | 7 | 8 | 8 | 8 | |
| | | | | | | | | | 1 | 3 | 1 | 5 | 4 | 7 | 6 | 5 | 3 | 7 | 6 | 9 | 6 | 8 | 6 | 8 | |
| 3 | | | | | | | | | 5 | 3 | 2 | 8 | 4 | 7 | 7 | 7 | 4 | 5 | 6 | 8 | 7 | 5 | 4 | 7 | 1 |
| | | | | | | | | | 1 | 3 | 1 | 7 | 3 | 5 | 5 | 6 | 3 | 2 | 4 | 8 | 5 | 4 | 4 | 6 | 1 |
| 6 | | | | | | | | | 5 | 4 | 2 | 7 | 6 | 6 | 6 | 8 | 5 | 8 | 6 | 9 | 8 | 7 | 6 | 8 | 2 |
| | | | | | | | | | 1 | 4 | 2 | 5 | 5 | 5 | 5 | 6 | 2 | 6 | 6 | 9 | 8 | 7 | 5 | 7 | 1 |
| 4 | 3 | | 3 | 6 | 4 | 4 | 4 | | 5 | 6 | 4 | 9 | 7 | 8 | 9 | 7 | 8 | 8 | 8 | 9 | 8 | 9 | 8 | 9 | |
| | | | | | | | | | 1 | 3 | 2 | 6 | 4 | 6 | 5 | 5 | 2 | 7 | 7 | 8 | 7 | 7 | 5 | 8 | |

Table III (Continued)

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 7 | | 2 | 6 | 4 | 6 | 4 | 6 | | 5 | 4 | 4 | 9 | 7 | 9 | 9 | 9 | 8 | 8 | 8 | 9 | 8 | 8 | 8 | 9 | 5 |
| | | | | | | | | | 1 | 3 | 2 | 6 | 4 | 7 | 8 | 7 | 8 | 6 | 6 | 9 | 7 | 8 | 6 | 8 | 2 |
| 5 | 2 | 1 | 3 | 6 | 3 | 2 | 2 | | 5 | 5 | 5 | 8 | 7 | 6 | 8 | 8 | 7 | 7 | 7 | 9 | 8 | 8 | 5 | 8 | 2 |
| | | | | | | | | | 1 | 2 | 3 | 6 | 5 | 5 | 6 | 6 | 4 | 5 | 6 | 8 | 6 | 7 | | 6 | 1 |
| 8 | | | | | | | | | 5 | 8 | 3 | 8 | 6 | 8 | 6 | 8 | 5 | 6 | 6 | 9 | 7 | 5 | 4 | 8 | 1 |
| | | | | | | | | | 1 | 5 | 3 | 7 | 6 | 8 | 6 | 6 | 4 | 5 | 5 | 8 | 6 | 4 | 4 | 7 | 1 |
| 9 | | | | | | | | | 5 | 3 | | 5 | 3 | 3 | 5 | 5 | 3 | | | | | | | | |
| | | | | | | | | | 1 | 1 | | 3 | | 3 | 4 | 4 | 2 | | | | | | | | |
| 10 | | | | | | | | | 5 | 4 | | 3 | 1 | | 4 | 6 | 5 | 6 | 6 | 8 | 7 | 4 | 2 | 8 | 3 |
| | | | | | | | | | 1 | 3 | | 3 | | | 3 | 4 | 4 | 4 | 6 | 8 | 7 | 4 | 2 | 8 | |

Table III (Continued)

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 11 | | 2 | 3 | 5 | 5 | 2 | 7 | 2 | 5 | 8 | 6 | 9 | 8 | 9 | 8 | 9 | 7 | 8 | 8 | 9 | 8 | 8 | 8 | 9 | 3 |
| | | | | | | | | | 1 | 7 | 4 | 8 | 6 | 8 | 6 | 6 | 6 | 7 | 7 | 9 | 7 | 7 | 6 | 8 | 1 |
| 12 | | | | | | | | | 5 | 3 | | 2 | | 3 | 3 | 4 | 3 | 3 | 3 | 8 | 4 | | 3 | 8 | 2 |
| | | | | | | | | | 1 | 3 | | 2 | | 2 | 2 | 2 | 3 | 3 | 3 | 8 | 4 | | 3 | 7 | 1 |
| 13 | | | 3 | 5 | 4 | 3 | 2 | | 5 | 5 | 3 | 8 | 6 | 6 | 6 | 7 | 3 | 6 | 6 | 9 | 7 | 6 | 5 | 8 | 2 |
| | | | | | | | | | 1 | 4 | 2 | 6 | 3 | 5 | 4 | 4 | 3 | 5 | 5 | 9 | 7 | 4 | 4 | 7 | 1 |
| 14 | | | | | | | | | 5 | 5 | 3 | 8 | 7 | 9 | 7 | 8 | 4 | 8 | 8 | 9 | 8 | 8 | 8 | 9 | 5 |
| | | | | | | | | | 1 | 4 | 2 | 8 | 6 | 8 | 7 | 7 | 3 | 7 | 8 | 9 | 7 | 8 | 6 | 9 | 5 |
| 15 | | | 3 | 3 | 3 | 4 | 3 | | 5 | 4 | 3 | 8 | 6 | 8 | 8 | 8 | 3 | 6 | 6 | 9 | 7 | 8 | 7 | 8 | 1 |
| | | | | | | | | | 1 | 3 | 3 | 7 | 6 | 7 | 7 | 7 | 2 | 5 | 5 | 8 | 5 | 6 | 5 | 8 | 1 |

Table III (Continued)

| Compound of Ex. No. | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | kg/ha | Mz | R | BG | O | L | M | SB | S | Mz | R | BG | O | L | M | SB | S |
| 16 | * | * | * | * | * | * | * | * | 5 | 2 | 1 | 7 | 5 | 6 | 9 | 6 | 4 | 4 | 5 | 8 | 5 | 6 | 7 | 8 | |
| | | | | | | | | | 1 | 2 | | 4 | 3 | 2 | 8 | 5 | 3 | 1 | | 7 | 3 | 4 | 4 | 8 | |
| 17 | | | | | | | | | 5 | 3 | 2 | 7 | 7 | 7 | 8 | 6 | 4 | 6 | 5 | 9 | 6 | 9 | 9 | 9 | 2 |
| | | | | | | | | | 1 | 2 | | 4 | 3 | 5 | 7 | 5 | 2 | 5 | 3 | 9 | 5 | 7 | 7 | 7 | 2 |
| 18 | | | | | | | | | 5 | | | 2 | 2 | 3 | 2 | 2 | | | | 5 | | | | | |
| | | | | | | | | | 1 | | | | | 1 | | | | | | | | | | | |
| 19 | | | 7 | 7 | 4 | | 3 | | 5 | 4 | 4 | 8 | 8 | 8 | 9 | 8 | 6 | 8 | 8 | 9 | 8 | 9 | 7 | 9 | 5 |
| | | | | | | | | | 1 | 4 | 2 | 7 | 6 | 9 | 7 | 6 | 3 | 6 | 6 | 9 | 6 | 8 | 5 | 8 | 2 |
| 20 | | | | | | | | | 5 | | | | 1 | 3 | 3 | 2 | 2 | 3 | 1 | 7 | 4 | 4 | 2 | 6 | 2 |
| | | | | | | | | | 1 | | | | | 1 | 1 | 2 | 1 | | | 7 | 2 | 3 | | 2 | |

Table III (Continued)

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 21 | | | 5 | 5 | 2 | | 4 | | 5 | 4 | 4 | 8 | 8 | 8 | 9 | 9 | 4 | 7 | 8 | 9 | 7 | 7 | 8 | 9 | 3 |
| | | | | | | | | | 1 | 2 | 1 | 8 | 6 | 7 | 8 | 8 | 4 | 4 | 2 | 8 | 5 | 6 | 7 | 8 | |
| 22 | | | | | | | | | 5 | 4 | 1 | 8 | 8 | 8 | 9 | 9 | 7 | 7 | 6 | 9 | 7 | 8 | 8 | 8 | 1 |
| | | | | | | | | | 1 | 4 | 1 | 7 | 7 | 7 | 6 | 7 | 4 | 7 | 4 | 9 | 6 | 7 | 6 | 8 | |
| 23 | 3 | 2 | 7 | 8 | 4 | 6 | 8 | 2 | 5 | 5 | 3 | 9 | 8 | 9 | 9 | 9 | 8 | 8 | 7 | 9 | 8 | 9 | 9 | 9 | 2 |
| | | | | | | | | | 1 | 4 | 2 | 8 | 7 | 8 | 9 | 8 | 8 | 8 | 6 | 9 | 7 | 8 | 7 | 8 | 2 |
| 24 | | | | | | | | | 5 | 4 | 1 | 8 | 6 | 7 | 7 | 7 | 5 | 8 | 7 | 9 | 7 | 9 | 8 | 9 | |
| | | | | | | | | | 1 | 2 | | 5 | 3 | 6 | 7 | 5 | 3 | 2 | 3 | 8 | 4 | 6 | 6 | 7 | |
| 25 | | | | | | | | | 5 | 3 | 1 | 8 | 7 | 7 | 8 | 8 | 6 | 8 | 7 | 9 | 8 | 8 | 8 | 8 | |
| | | | | | | | | | 1 | 3 | 1 | 6 | 4 | 6 | 8 | 6 | 6 | 8 | 7 | 9 | 7 | | 6 | 7 | |

Table III (Continued)

| Compound of Ex. No. | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | kg/ha | Mz | R | BG | O | L | M | SB | S | Mz | R | BG | O | L | M | SB | S |
| 26 | | | | | | | | | 5 | 3 | 1 | 9 | 7 | 8 | 8 | 9 | 7 | 8 | 8 | 9 | 8 | 8 | 8 | 9 | 2 |
| | | | | | | | | | 1 | 2 | 1 | 7 | 5 | 6 | 8 | 8 | 5 | 8 | 8 | 9 | 7 | 7 | 7 | 9 | |
| 27 | | | | | | | | | 5 | 6 | 4 | 8 | 6 | 9 | 6 | 7 | 5 | 8 | 8 | 9 | 8 | 8 | 5 | 9 | |
| | | | | | | | | | 1 | 4 | 2 | 6 | 5 | 6 | 4 | 6 | 4 | 8 | 8 | 9 | 6 | 6 | 5 | | |
| 28 | | | 4 | 4 | | 3 | 4 | | 5 | 7 | 3 | 9 | 8 | 8 | 9 | 8 | 7 | 7 | 7 | 9 | 8 | 8 | 8 | 8 | 1 |
| | | | | | | | | | 1 | 6 | 3 | 9 | 7 | 7 | 5 | 8 | 5 | 7 | 7 | 9 | 7 | 7 | 7 | 7 | |
| 29 | | | 4 | 1 | 2 | 3 | 4 | | 5 | 5 | 4 | 7 | 5 | 9 | 8 | 9 | 4 | 8 | 8 | 9 | 8 | 8 | 8 | 9 | 6 |
| | | | | | | | | | 1 | 3 | 2 | 6 | 4 | 7 | 7 | 7 | 2 | 8 | 8 | 9 | 8 | 6 | 7 | 8 | 3 |
| 30 | 2 | 2 | 6 | 6 | 6 | 4 | 6 | 2 | 5 | 7 | 3 | 9 | 8 | 9 | 8 | 8 | 5 | 8 | 8 | 9 | 8 | 8 | 8 | 9 | 1 |
| | | | | | | | | | 1 | 6 | 3 | 8 | 7 | 9 | 8 | 7 | 5 | 8 | 8 | 9 | 8 | 8 | 7 | 8 | |

Table III (Continued)

| Compound of Ex. No. | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | kg/ha | Mz | R | BG | O | L | M | SB | S | Mz | R | BG | O | L | M | SB | S |
| 31 | | 1 | 6 | 4 | 2 | 2 | 4 | | 5 | 6 | 4 | 8 | 8 | 9 | 9 | 8 | 6 | 7 | 8 | 9 | 8 | 7 | 8 | 9 | 2 |
| | | | | | | | | | 1 | 4 | 2 | 6 | 5 | 8 | 8 | 6 | 4 | 5 | 8 | 9 | 6 | 6 | 7 | 8 | |
| 32 | | | 4 | 6 | 3 | 3 | 3 | | 5 | 7 | 4 | 9 | 8 | 9 | 9 | 9 | 5 | 8 | 8 | 9 | 8 | 8 | 8 | 9 | |
| | | | | | | | | | 1 | 5 | 2 | 8 | 6 | 8 | 9 | 8 | 5 | 7 | 8 | 9 | 7 | 7 | 7 | 8 | |
| 33 | | | | | | | | | 5 | 5 | 3 | 6 | 4 | 7 | 7 | 5 | 3 | 8 | 7 | 9 | 7 | 6 | 5 | 7 | |
| | | | | | | | | | 1 | 4 | 2 | 4 | 2 | 6 | 4 | 4 | 2 | 8 | 6 | 8 | 7 | 6 | 5 | 7 | |
| 34 | | | 5 | 4 | 5 | 4 | 6 | 2 | 5 | 6 | 4 | 8 | 7 | 9 | 8 | 7 | 6 | 8 | 7 | 9 | 8 | 8 | 7 | 8 | |
| | | | | | | | | | 1 | 4 | 3 | 6 | 5 | 7 | 6 | 7 | 4 | 8 | 7 | 9 | 6 | 7 | 6 | 8 | |
| 35 | | | 5 | 4 | 6 | 3 | 6 | 2 | 5 | 5 | 3 | 6 | 3 | 8 | 8 | 6 | 6 | 7 | 4 | 8 | 6 | 7 | 7 | 8 | |
| | | | | | | | | | 1 | 3 | | 5 | 3 | 7 | 6 | 5 | 4 | 3 | 2 | 7 | 4 | 5 | 6 | 7 | |

Table III (Continued)

| Compound of | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 36 | | | | | 6 | 4 | 5 | 3 | 5 | 3 | | 7 | 4 | 8 | 6 | 7 | 5 | 4 | 4 | 8 | 5 | 6 | 8 | 8 | |
| | | | | | | | | | 1 | 2 | | 4 | 3 | 7 | 5 | 6 | 4 | 4 | 4 | 8 | 4 | 6 | 7 | 8 | |
| 37 | 2 | 2 | 7 | 6 | 4 | 3 | 6 | 2 | 5 | 6 | 4 | 9 | 7 | 9 | 7 | 8 | 7 | 8 | 8 | 9 | 8 | 8 | 8 | 8 | |
| | | | | | | | | | 1 | 5 | 3 | 7 | 5 | 8 | 5 | 7 | 5 | 6 | 6 | 9 | 6 | 5 | 4 | 8 | |
| 38 | 4 | 3 | 7 | 7 | 6 | 4 | 7 | 4 | 5 | 6 | 3 | 6 | 7 | 7 | 6 | 6 | 4 | 8 | 8 | 9 | 8 | 7 | 7 | 8 | 3 |
| | | | | | | | | | 1 | 4 | | 4 | 5 | 5 | 4 | 4 | 4 | 7 | 7 | 9 | 7 | 7 | 6 | 8 | 3 |
| 39 | | | | | | | | | 5 | 3 | 2 | 4 | 4 | 7 | 6 | 6 | 4 | | | | | 4 | 5 | 8 | |
| | | | | | | | | | 1 | * | * | * | * | * | * | * | * | * | * | * | * | * | * | * | * |
| 40 | | | 8 | 5 | 3 | 3 | 5 | | 5 | 7 | 6 | 9 | 8 | 9 | 9 | 9 | 8 | 8 | 7 | 9 | 8 | 8 | 8 | 8 | 1 |
| | | | | | | | | | 1 | 6 | 4 | 8 | 6 | 9 | 8 | 9 | 6 | 6 | 6 | 8 | 6 | 6 | 7 | 8 | |

Table III (Continued)

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 41 | | | | | | | | | 5 | 1 | | 5 | 1 | 6 | 4 | 3 | 3 | 7 | 7 | 9 | 9 | 8 | 8 | 9 | |
| | | | | | | | | | 1 | 1 | | 1 | 1 | 5 | 3 | 2 | 3 | 7 | 7 | 9 | 8 | 8 | 7 | 9 | |
| 42 | | | | | | | | | 5 | 1 | | 2 | 1 | 4 | 4 | 1 | 3 | | 3 | 8 | 5 | 5 | 6 | 8 | |
| | | | | | | | | | 1 | | | | | 4 | 2 | | | | 2 | 1 | 2 | 2 | | | |
| 43 | | | | | | | | | 5 | 7 | 4 | 6 | 6 | 7 | 6 | 6 | 4 | 5 | 6 | 8 | 6 | 7 | 3 | 8 | |
| | | | | | | | | | 1 | 4 | 2 | 4 | 5 | 7 | 5 | 5 | 4 | | 4 | 7 | 5 | 5 | 2 | 6 | |
| 44 | | | 6 | 3 | 4 | | 4 | 3 | 5 | 6 | 6 | 9 | 8 | 8 | 7 | 8 | 5 | 8 | 9 | 9 | 8 | 8 | 7 | 8 | 4 |
| | | | | | | | | | 1 | 4 | 4 | 8 | 7 | 7 | 6 | 7 | 5 | 7 | 8 | 9 | 8 | 8 | 7 | 8 | 2 |
| 45 | | | 6 | 5 | 4 | | 4 | | 5 | 6 | 4 | 9 | 7 | 8 | 7 | 8 | 5 | 7 | 9 | 9 | 8 | 8 | 8 | 8 | 3 |
| | | | | | | | | | 1 | 4 | 2 | 7 | 6 | 7 | 5 | 6 | 3 | 7 | 9 | 9 | 8 | 8 | 6 | 8 | 1 |

Table III (Continued)

| Compound of Ex. No. | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | kg/ha | Mz | R | BG | O | L | M | SB | S | Mz | R | BG | O | L | M | SB | S |
| 46 | | | 8 | 4 | 2 | | 2 | | 5 | 7 | 3 | 9 | 7 | 9 | 7 | 8 | 6 | 8 | 9 | 9 | 9 | 9 | 8 | 9 | 5 |
| | | | | | | | | | 1 | 5 | 2 | 7 | 5 | 8 | 4 | 7 | 4 | 8 | 8 | 9 | 8 | 9 | 8 | 8 | 2 |
| 47 | | | 4 | 6 | 3 | 6 | 5 | | 5 | 8 | 4 | 8 | 8 | 9 | 9 | 9 | 9 | 8 | 8 | 9 | 7 | 9 | 9 | 9 | |
| | | | | | | | | | 1 | 6 | 4 | 7 | 6 | 8 | 8 | 7 | 8 | 6 | 7 | 9 | 7 | 8 | 8 | 8 | |
| 48 | 4 | 2 | 8 | 8 | 4 | 5 | 7 | | 5 | 8 | 5 | 9 | 9 | 9 | 9 | 9 | 7 | 9 | 9 | 9 | 9 | 9 | 9 | 9 | 4 |
| | | | | | | | | | 1 | 7 | 3 | 7 | 7 | 9 | 8 | 7 | 6 | 7 | 7 | 9 | 7 | 8 | 7 | 8 | |
| 49 | | | | | | | | | 5 | 6 | 3 | 8 | 7 | 9 | 7 | 7 | 5 | 6 | 8 | 9 | 9 | 9 | 8 | 9 | 3 |
| | | | | | | | | | 1 | 2 | 1 | 5 | 5 | 8 | 7 | 5 | 5 | 5 | 6 | 9 | 8 | 8 | 8 | 8 | 2 |
| 50 | | | | | | | | | 5 | 3 | 2 | 7 | 5 | 8 | 6 | 7 | 4 | 4 | 5 | 9 | 8 | 7 | 6 | 8 | 1 |
| | | | | | | | | | 1 | 2 | 2 | 4 | 4 | 7 | 6 | 2 | 3 | 1 | 2 | 8 | 5 | 6 | 6 | 8 | |

Table III (Continued)

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 51 | | | 1 | | 2 | | 4 | 1 | 5 | 7 | 5 | 9 | 9 | 9 | 8 | 9 | 7 | 8 | 8 | 9 | 9 | 9 | 9 | 9 | 6 |
| | | | | | | | | | 1 | 5 | 4 | 8 | 7 | 9 | 7 | 6 | 7 | 6 | 7 | 9 | 8 | 8 | 7 | 8 | 2 |
| 52 | | | 5 | 6 | 3 | | 5 | 1 | 5 | 8 | 6 | 9 | 9 | 9 | 9 | 9 | 9 | 7 | 8 | 9 | 8 | 9 | 8 | 9 | 7 |
| | | | | | | | | | 1 | 6 | 4 | 8 | 8 | 9 | 8 | 9 | 6 | 5 | 7 | 8 | 7 | 8 | 7 | 8 | 3 |
| 53 | | | 6 | 7 | 4 | 1 | 5 | 1 | 5 | 6 | 4 | 9 | 7 | 9 | 8 | 8 | 4 | 7 | 7 | 9 | 8 | 9 | 8 | 8 | 2 |
| | | | | | | | | | 1 | 5 | 3 | 7 | 5 | 8 | 7 | 7 | 3 | 6 | 5 | 9 | 7 | 8 | 6 | 8 | 1 |
| 54 | | | | | | | | | 5 | | | | | 3 | 6 | 5 | 5 | | | | | | | | |
| | | | | | | | | | 1 | | | | | 2 | 3 | 3 | 3 | | | | | | | | |
| 55 | | | | 3 | 3 | | 6 | 2 | 5 | 5 | 4 | 5 | 5 | 6 | 7 | 7 | 5 | | | 7 | 6 | 6 | 6 | 8 | 2 |
| | | | | | | | | | 1 | 5 | 3 | 4 | 4 | 5 | 5 | 6 | 3 | | | 7 | 4 | 5 | 5 | 7 | 1 |

**Table III (Continued)**

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 73 | 3 | 4 | 8 | 8 | 6 | 4 | 3 | 3 | 5 | 7 | 6 | 9 | 9 | 9 | 9 | 9 | 8 | 8 | 9 | 9 | 9 | 9 | 9 | 9 | 3 |
| | | | | | | | | | 1 | 4 | 4 | 7 | 7 | 9 | 8 | 7 | 6 | 6 | 8 | 9 | 8 | 8 | 8 | 8 | 1 |
| 74 | 3 | 3 | 6 | 7 | 7 | 6 | 6 | 2 | 5 | 7 | 5 | 7 | 7 | 9 | 8 | 8 | 7 | 6 | 6 | 9 | 7 | 8 | 9 | 8 | 2 |
| | | | | | | | | | 1 | * | * | * | * | * | * | * | * | * | * | * | * | * | * | * | * |
| 69 | | | | | | | | | 5 | 7 | 5 | 8 | 7 | 9 | 8 | 8 | 7 | 6 | 6 | 8 | 6 | 7 | 6 | 9 | 2 |
| | | | | | | | | | 1 | 5 | 3 | 7 | 5 | 9 | 6 | 8 | 5 | 4 | 4 | 8 | 5 | 5 | 4 | 7 | 1 |
| 70 | | | | | | | | | 5 | 5 | | 4 | 3 | 8 | 7 | 6 | 4 | 3 | 3 | 8 | 5 | 5 | 4 | 8 | |
| | | | | | | | | | 1 | 2 | | 2 | 1 | 7 | 4 | 4 | 3 | 1 | 1 | 7 | 3 | 5 | 3 | 7 | |
| 56 | | | 3 | 7 | 6 | 3 | 5 | | 5 | 8 | 4 | 8 | 8 | 9 | 7 | 8 | 5 | 8 | 8 | 9 | 8 | 8 | 7 | 9 | 4 |
| | | | | | | | | | 1 | 6 | 4 | 8 | 6 | 9 | 7 | 6 | 4 | 8 | 8 | 9 | 7 | 7 | 6 | 8 | 1 |

Table III (Continued)

| Compound of | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 57 | | | | | | | 4 | | 5 | 6 | 5 | 8 | 7 | 9 | 7 | 7 | 6 | 7 | 6 | 9 | 7 | 7 | 6 | 8 | |
| | | | | | | | | | 1 | 4 | 3 | 6 | 5 | 7 | 4 | 5 | 4 | 6 | 6 | 9 | 7 | 7 | 5 | 8 | |
| 58 | | | | | | | 4 | | 5 | 4 | 3 | 5 | 5 | 8 | 6 | 7 | 6 | 6 | 6 | 9 | 8 | 8 | 8 | 8 | |
| | | | | | | | | | 1 | 2 | 1 | 3 | 3 | 7 | 5 | 5 | 4 | 5 | 5 | 9 | 8 | 8 | 7 | 8 | |
| 59 | | | | | 2 | | 3 | | 5 | 7 | 3 | 5 | 4 | 7 | 5 | 5 | 4 | 8 | 8 | 9 | 8 | 8 | 5 | 8 | |
| | | | | | | | | | 1 | 3 | 2 | 3 | 2 | 6 | 4 | 5 | 4 | 6 | 6 | 9 | 7 | 6 | 3 | 7 | |
| 60 | | | 3 | 7 | 7 | | 6 | 3 | 5 | 4 | 4 | 9 | 8 | 9 | 8 | 8 | 6 | 8 | 9 | 9 | 9 | 9 | 8 | 9 | 4 |
| | | | | | | | | | 1 | 4 | 2 | 7 | 6 | 8 | 6 | 7 | 5 | 8 | 8 | 9 | 8 | 7 | 6 | 9 | 1 |
| 61 | | | | | | | | | 5 | 5 | 3 | 7 | 6 | 9 | 7 | 7 | 7 | 7 | 6 | 9 | 7 | 6 | 4 | 8 | |
| | | | | | | | | | 1 | 4 | 1 | 4 | 4 | 7 | 5 | 5 | 5 | 4 | 6 | 8 | 7 | 6 | 2 | 8 | |

**Table III (Continued)**

| Compound of Ex. No. | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | kg/ha | Mz | R | BG | O | L | M | SB | S | Mz | R | BG | O | L | M | SB | S |
| 62 | | | 6 | 5 | 4 | 2 | 5 | | 5 | 2 | 3 | 6 | 6 | 8 | 5 | 6 | 5 | 7 | 7 | 9 | 8 | 7 | 6 | 9 | 2 |
| | | | | | | | | | 1 | 1 | 2 | 3 | 3 | 6 | 4 | 6 | 4 | 4 | 5 | 8 | 7 | 2 | 1 | 7 | |
| 63 | | | 3 | 2 | 1 | | | | 5 | 2 | 2 | 3 | 2 | 5 | 3 | 2 | 3 | 7 | 7 | 9 | 8 | 5 | 5 | 8 | 2 |
| | | | | | | | | | 1 | 2 | 1 | 2 | 1 | 5 | 2 | 2 | 2 | 5 | 3 | 8 | 4 | 2 | 1 | 7 | |
| 64 | | | | | | | | | 5 | 3 | 2 | 3 | 2 | 5 | 4 | 3 | 4 | 5 | 5 | 9 | 6 | 4 | 2 | 8 | |
| | | | | | | | | | 1 | 3 | 1 | 2 | 2 | 4 | 4 | 2 | 2 | 5 | 3 | 9 | 5 | 2 | 1 | 6 | |
| 65 | | | | | | | | | 5 | 6 | 4 | 6 | 5 | 6 | 4 | 5 | 6 | 7 | 5 | 8 | 7 | 3 | 4 | 5 | |
| | | | | | | | | | 1 | 3 | 2 | 3 | 3 | 4 | 4 | 4 | 4 | 1 | 2 | 6 | 4 | 1 | 2 | 3 | |
| 66 | | | 4 | 7 | 5 | | | | 5 | 5 | 5 | 8 | 8 | 9 | 7 | 7 | 7 | 6 | 8 | 9 | 8 | 7 | 6 | 8 | |
| | | | | | | | | | 1 | 3 | 3 | 5 | 6 | 8 | 5 | 5 | 6 | 4 | 5 | 8 | 6 | 3 | 3 | 6 | |

Table III (Continued)

| Compound of Ex. No. | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | kg/ha | Mz | R | BG | O | L | M | SB | S | Mz | R | BG | O | L | M | SB | S |
| 71 | 3 | 4 | 4 | 6 | 6 | 3 | 5 | 2 | 5 | 7 | 6 | 9 | 9 | 9 | 9 | 9 | 9 | 7 | 6 | 9 | 8 | 8 | 7 | 9 | |
| | | | | | | | | | 1 | 6 | 4 | 7 | 8 | 9 | 8 | 9 | 7 | 3 | 4 | 8 | 7 | 6 | 4 | 8 | |
| 72 | 3 | 4 | 6 | 7 | 8 | 7 | 6 | 1 | 5 | 7 | 5 | 9 | 9 | 9 | 9 | 9 | 9 | 8 | 8 | 9 | 9 | 8 | 9 | 9 | 3 |
| | | | | | | | | | 1 | 7 | 3 | 6 | 7 | 9 | 8 | 8 | 6 | 6 | 6 | 9 | 7 | 6 | 8 | 8 | |
| 75 | 5 | 4 | 4 | 6 | 5 | 5 | 6 | 2 | 5 | 5 | 5 | 6 | 5 | 9 | 7 | 7 | 6 | | 2 | 8 | 7 | 6 | 8 | 8 | |
| | | | | | | | | | 1 | 4 | 4 | 4 | 4 | 8 | 6 | 6 | 6 | | 1 | 7 | 4 | 3 | 6 | 7 | |
| 67 | | 2 | 4 | 4 | | | | | 5 | 1 | | 5 | 5 | 8 | 6 | 6 | 5 | 7 | 6 | 9 | 7 | 5 | 4 | 8 | |
| | | | | | | | | | 1 | 1 | | 2 | 2 | 7 | 3 | 3 | 2 | | | 8 | 3 | 1 | | 4 | |
| 68 | 2 | 3 | 4 | 5 | 5 | | 2 | | 5 | 2 | 3 | 7 | 6 | 9 | 6 | 9 | 5 | | 1 | 8 | 3 | 6 | 5 | 7 | |
| | | | | | | | | | 1 | | | | | | | | | | | | | | | | |

**Claims for the contracting states: BE, CH, DE, FR, GB, IT, LI, LU, NL, SE**

1. Hydrazino diphenyl ethers having the general formula I:-

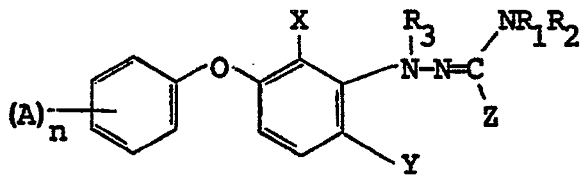

I

wherein: n is 0, 1,2 or 3; each group A, which may be the same or different when n is greater than 1, independently represents a halogen atom or a cyano, nitro, alkyl or haloalkyl group;
X represents a hydrogen atom or, when n is 0, alternatively represents a halogen atom;
Y represents a halogen atom or a nitro or cyano group; $R_1$ represents a hydrogen atom, an alkyl, cycloalkyl, alkenyl or alkynyl group, an alkyl group substituted by a halogen atom or a cyano or alkoxy group, or an optionally substituted aryl group;
$R_2$ represents a hydrogen atom, an alkyl, alkoxy, cycloalkyl, alkenyl or alkynyl group, an alkyl group substituted by a halogen atom or a cyano, alkoxy, alkylamino, acylamino, alkoxycarbonyl or acyl group, an optionally substituted aryl or heterocyclic group, or an amino group optionally substituted by an alkyl, aryl, alkanoyl, aroyl or alkoxycarbonyl group or $R_1$ and $R_2$ together with the interjacent nitrogen atom jointly represent an azido group or a heterocyclic ring;
Z represents a hydrogen atom or a cyano, alkyl, alkylamino, carboxy, alkoxycarbonyl, or acyl group, or Z, $R_1$ and $R_2$, together with the interjacent carbon and nitrogen atoms represent a heterocyclic ring;
and $R_3$ represents a hydrogen atom or an alkyl group.

2. Compounds as claimed in claim 1, wherein one substituent A is located para to the ether linkage and the other substituents, when present, are located ortho to the ether linkage.

3. Compounds as claimed in claim 1 or 2 wherein n is 2 or 3; one group A is a haloalkyl group, the second group A is a halogen atom or a nitro or cyano group, and third group A, when present, is a halogen atom.

4. Compounds as claimed in claim 3 wherein the haloalkyl group is a trifluoromethyl group and the halogen atom is a chlorine atom.

5. Compounds as claimed in any one of claims 1-4, wherein X represents a hydrogen atom, Y represents a nitro group, and $R_3$ represents a hydrogen atom or an alkyl group of up to 6 carbon atoms.

6. Compounds as claimed in any one of the preceding claims, wherein $R_1$ is a hydrogen atom or an optionally chlorinated alkyl group of up to 8 carbon atoms; and Z is a hydrogen atom or a methyl, cyano, carboxyl, alkylamino, or phenylsulphonyl group; an alkoxycarbonyl or aralkoxycarbonyl group; an alkanoyl group; or an N-substituted carbamoyl group of the formula $CONR_4R_5$, wherein $R_4$ represents a hydrogen atom or an alkyl group of up to 6 carbon atoms, and $R_5$ represents an alkyl group of up to 8 carbon atoms, optionally bearing an alkoxy or alkylamino substituent; a cyclopropyl group; or an amino group optionally bearing an alkyl, alkenyl or alkynyl substituent.

7. Compounds as claimed in any of the preceding claims, wherein $R_2$ is a hydrogen atom, an alkyl group of up to 8 carbon atoms optionally bearing a halogen or alkoxy substituent; a cyclopropyl or cyclohexyl group, optionally bearing an alkoxycarbonyl substituent; an alkenyl or alkynyl group of up to 8 carbon atoms; a methyl or ethyl group bearing an alkoxycarbonyl, carbamoyl or alkylamino group; a phenyl group; an alkoxy group; a thiophene ring, optionally bearing an alkoxycarbonyl substituent; or an amino group optionally substituted by an alkyl, phenyl, acetyl, benzoyl or alkoxycarbonyl group.

8. Compounds as claimed in any one of claims 1-6, wherein $R_1$ and $R_2$, together with the interjacent nitrogen atom, represent an azido group or a nitrogen-containing heterocyclic ring selected from pyrrolidine, piperidine, hydro-oxazine or triazole; or $R_1$, $R_2$ and Z, together with the interjacent nitrogen and carbon atoms, represent a diazole or hydrodiazine ring.

9. Process for the preparation of a compound of the general formula I as defined in claim 1, which comprises reacting an amine of general formula $HNR_1R_2$ with a hydrazonyl halide of general fomula II:

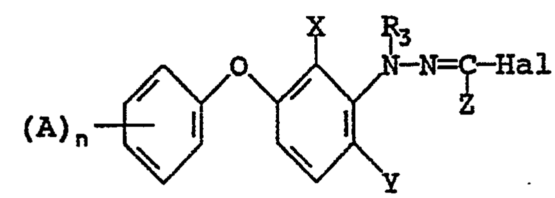

II

wherein Hal represents a halogen atom; and the other substituents have the meanings defined in claim 1.

10. Process as claimed in claim 9 wherein Hal represents a chlorine atom and the reaction is carried out under reflux.

11. Process as claimed in claim 9 or 10 wherein the starting hydrazonyl halide of formula II in which Z is other than alkyl or hydrogen, is prepared by diazotization of an aniline derivative of formula III:

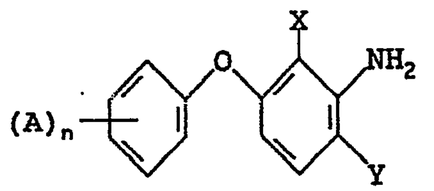

III

wherein n, A, X and Y are as defined in claim 1, and coupling with a haloacyl compound of general formula IV:-

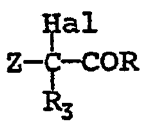

IV

wherein Hal represents a halogen atom, R represents an alkyl group, and $R_3$ is as defined in claim 1.

12. Process as claimed in claim 11 wherein the haloacyl compound has the formula $Z\text{-}CR_3Cl\text{-}COCH_3$.

13. Process as claimed in claim 11 or 12 wherein Z in formula IV represents an alkoxycarbonyl group, and the resultant hydrazonyl halide of formula II is reacted with an excess of amine to yield a compound of formula I wherein Z represents an alkylcarbamoyl group.

14. Process as claimed is claimed 11 or 12 wherein Z in formula IV represents a sulphonyl group, and the resulting hydrazonyl halide of formula II wherein Z is sulphonyl is reacted with an excess of amine to yield a compound of formula I wherein Z represents an alkylamino group.

15. Process as claimed in any one of claims 11-14 wherein the diazotization is carried out at a temperature between 0° and 10°C.

16. Process as claimed in claim 9 or 10 wherein the starting hydrazonyl halide of formula II in which Z is alkyl or hydrogen is prepared by reacting a hydrazine of formula $R_3NH\text{-}NH_2$ with a nitro compound of formula V:

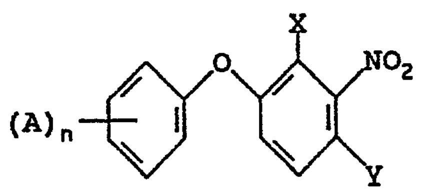

V

acylating the resulting phenyl hydrazine by reaction with an acid, its anhydride, or an acyl halide of formula ZCOOH or ZCOHal, wherein Z is hydrogen or alkyl and Hal represents a halogen atom, followed by reaction with phosphorus pentachloride.

17. Process as claimed in claim 16 wherein the reaction of the nitro and hydrazine compounds, and the subsequent acylation, are each carried out in an inert solvent.

18. Process as claimed in claim 16 or 17 wherein the acylation is carried out in the presence of a hydrogen halide acceptor.

19. Herbicidal composition, which comprises a compound as claimed in any of claims 1 to 8, together with a carrier.

20. A composition as claimed in claim 19, which comprises at least two carriers, at least one of which is a surface-active agent.

21. Method of combating undesired plant growth at a locus, which comprises treating the locus with a compound as claimed in any one of claims 1 to 8 or a composition as claimed in either of claims 19 and 20.

22. The use of a compound as claimed in any one of claims 1 to 8 as a herbicide.

**Claims for the Contracting State: AT**

1. Herbicidal composition, which comprises a hydrazino diphenyl ether having the general formula I:

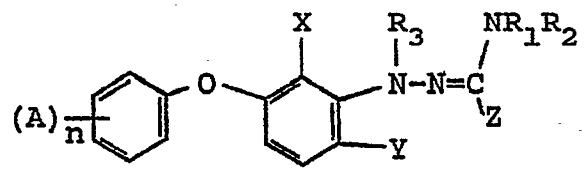

I

wherein: n is 0, 1, 2 or 3; each group A, which may be the same or different when n is greater than 1, independently represents a halogen atom or a cyano, nitro, alkyl or haloalkyl group;
X represents a hydrogen atom or, when n is 0, alternatively represents a halogen atom;
Y represents a halogen atom or a nitro or cyano group;
$R_1$ represents a hydrogen atom, an alkyl, cycloalkyl, alkenyl or alkynyl group, an alkyl group substituted by a halogen atom or a cyano or alkoxy group, or an optionally substituted aryl group;
$R_2$ represents a hydrogen atom, an alkyl, alkoxy, cycloalkyl, alkenyl or alkynyl group, an alkyl group substituted by a halogen atom or a cyano, alkoxy, alkylamino, acylamino, alkoxycarbonyl or acyl group, an optionally substituted aryl or heterocyclic group, or an amino group optionally substituted by an alkyl, aryl, alkanoyl, aroyl or alkoxycarbonyl group or $R_1$ and $R_2$ together with the interjacent nitrogen atom jointly represent an azido group or a heterocyclic ring;
Z represents a hydrogen atom or a cyano, alkyl, alkylamino, carboxy, alkoxycarbonyl, or acyl group, or Z, $R_1$ and $R_2$, together with the interjacent carbon and nitrogen atoms represent a heterocyclic ring; and
$R_3$ represents a hydrogen atom or an alkyl group;
together with a carrier.

2. A composition as claimed in claim 1, wherein, in the general formula I, one substituent A is located para to the ether linkage and the other substituents, when present, are located ortho to the ether linkage.

3. A composition as claimed in claim 1 or 2, wherein, in the general formula I, n is 2 or 3; one group A is a haloalkyl group, the second group A is a halogen atom or a nitro or cyano group, and third group A, when present, is a halogen atom.

4. A composition as claimed in claim 3, wherein, in the general formula I, the haloalkyl group is a trifluoromethyl group and the halogen atom is a chlorine atom.

5. A composition as claimed in any one of claims 1-4, wherein, in the general formula I, X represents a hydrogen atom, Y represents a nitro group, and $R_3$ represents a hydrogen atom or an alkyl group of up to 6 carbon atoms.

6. A composition as claimed in any one of the preceding claims, wherein, in the general formula I, $R_1$ is a hydrogen atom or an optionally chlorinated alkyl group of up to 8 carbon atoms; and Z is a hydrogen atom or a methyl, cyano, carboxyl, alkylamino, or phenylsulphonyl group; an alkoxycarbonyl or aralkoxycarbonyl group; an alkanoyl group; or an N-substituted carbamoyl group of the formula $CONR_4R_5$, wherein $R_4$ represents a hydrogen atom or an alkyl group of up to 6 carbon atoms, and $R_5$ represents an alkyl group of up to 8 carbon atoms, optionally bearing an alkoxy or alkylamino substituent; a cyclopropyl group; or an amino group optionally bearing an alkyl, alkenyl or alkynyl substituent.

7. A composition as claimed in any of the preceding claims, wherein, in the general formula I, $R_2$ is a hydrogen atom, an alkyl group of up to 8 carbon atoms optionally bearing a halogen or alkoxy substituent; a cyclopropyl or cyclohexyl group, optionally bearing an alkoxycarbonyl substituent; an alkenyl or alkynyl group of up to 8 carbon atoms; a methyl or ethyl group bearing an alkoxycarbonyl, carbamoyl or alkylamino group; a phenyl group; an alkoxy group; a thiophene ring, optionally bearing an alkoxycarbonyl substituent; or an amino group optionally substituted by an alkyl, phenyl, acetyl, benzoyl or alkoxycarbonyl group.

8. A composition as claimed in any one of the claims 1-6, wherein, in general formula I, $R_1$ and $R_2$, together with the interjacent nitrogen atom, represent an azido group or a nitrogen-containing heterocyclic ring selected from pyrrolidine, piperidine, hydro-oxazine or triazole; or $R_1$, $R_2$ and Z, together with the interjacent nitrogen and carbon atoms, represent a diazole or hydrodiazine ring.

9. A composition as claimed in any one of claims 1-8, which comprises at least two carriers, at least one of which is a surface-active agent.

10. Process for the preparation of a compound of the general formula I as defined in claim 1, which comprises reacting an amine of general formula $HNR_1R_2$ with a hydrazonyl halide of general formula II:

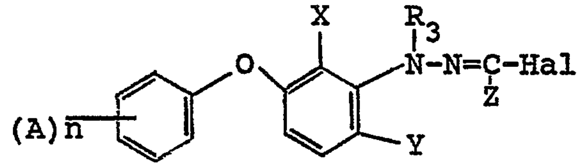

II

wherein Hal represents a halogen atom; and the other substituents have the meanings defined in claim 1.

11. Process as claimed in claim 10, wherein Hal represents a chlorine atom and the reaction is carried out under reflux.

12. Process as claimed in claim 10 or 11, wherein the starting hydrazonyl halide of formula II in which Z is other than alkyl or hydrogen, is prepared by diazotization of an aniline derivative of formula III:

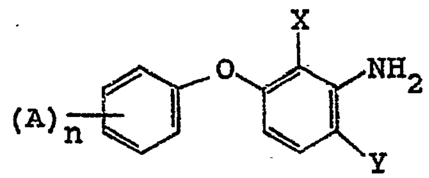

III

wherein n, A, X and Y are as defined in claim 1, and coupling with a haloacyl compound of general formula IV:

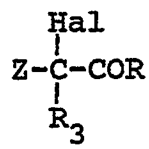

IV

wherein Hal represents a halogen atom, R represents an alkyl group, and $R_3$ is as defined in claim 1.

13. Process as claimed in claim 12, wherein the haloacyl compound has the formula Z-$CR_3Cl$-$COCH_3$.

14. Process as claimed in claim 12 or 13, wherein Z in formula IV represents an alkoxycarbonyl group, and the resultant hydrazonyl halide of formula II is reacted with an excess of amine to yield a compound of formula I wherein Z represents an alkylcarbamoyl group.

15. Process as claimed is claimed 12 or 13, wherein Z in formula IV represents a sulphonyl group, and the resulting hydrazonyl halide of formula II wherein Z is sulphonyl is reacted with an excess of amine to yield a compound of formula I wherein Z represents an alkylamino group.

16. Process as claimed in any one of claims 12-15, wherein the diazotization is carried out at a temperature between 0° and 10°C.

17. Process as claimed in claim 10 or 11, wherein the starting hydrazonyl halide of formula II in which Z is alkyl or hydrogen is prepared by reacting a hydrazine of formula $R_3NH$-$NH_2$ with a nitro compound of formula V:

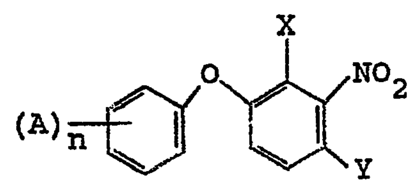

V

acylating the resulting phenyl hydrazine by reaction with an acid, its anhydride, or an acyl halide of formula ZCOOH or ZCOHal, wherein Z is hydrogen or alkyl and Hal represents a halogen atom, followed by reaction with phosphorus pentachloride.

18. Process as claimed in claim 17, wherein the reaction of the nitro and hydrazine compounds, and the subsequent acylation, are each carried out in an inert solvent.

19. Process as claimed in claim 17 or 18, wherein the acylation is carried out in the presence of a hydrogen halide acceptor.

20. Method of combating undesired plant growth at a locus, which comprises treating the locus with a compound as defined in any one of claims 1 to 8 or a composition as claimed in any one of claims 1 to 9.

21. The use of a compound as defined in any one of claims 1 to 8 as a herbicide.

**Patentansprüche für die Vertragsstaaten: BE, CH, DE, FR, GB, IT, LI, LU, NL, SE**

1. Hydrazinodiphenylether mit der allgemeinen Formel I:

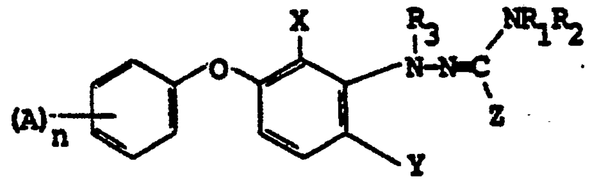

I

worin

n den Wert 0, 1, 2 oder 3 aufweist; jede Gruppe A, die gleich oder verschieden sein kann, wenn n größer als 1 ist, unabhängig voneinander ein Halogenatom oder eine Cyano-, Nitro-, Alkyl- oder Halogenalkylgruppe darstellt;

X ein Wasserstoffatom bedeutet, oder, wenn n den Wert 0 aufweist, in alternativer Weise ein Halogenatom bedeuten kann;

Y ein Halogenatom darstellt, oder eine Cyanogruppe oder Nitrogruppe bedeutet;

$R_1$ ein Wasserstoffatom, eine Alkyl-, Cycloalkyl-, Alkenyl- oder Alkinylgruppe, eine durch ein Halogenatom oder eine Cyano- oder Alkoxygruppe substituierte Alkylgruppe oder eine gegebenenfalls substituierte Arylgruppe darstellt;

$R_2$ ein Wasserstoffatom, eine Alkyl-, Alkoxy-, Cycloalkyl-, Alkenyl- oder Alkinylgruppe, eine durch ein Halogenatom oder eine Cyano-, Alkoxy-, Alkylamino-, Acylamino-, Alkoxycarbonyl- oder Acylgruppe substituierte Alkylgruppe, eine gegebenenfalls substituierte Aryl- oder heterocyclische Gruppe oder eine Aminogruppe, die gegebenenfalls durch eine Alkyl-, Aryl-, Alkanoyl-, Aroyl- oder Alkoxycarbonylgruppe substituiert ist, darstellt oder $R_1$ und $R_2$ zusammen mit dem dazwischenliegenden Stickstoffatom eine Azidogruppe oder einen heterocyclischen Ring bedeuten;

Z ein Wasserstoffatom oder eine Cyano-, Alkyl-, Alkylamino-, Carboxy-, Alkoxycarbonyl- oder Acylgruppe darstellt oder Z, $R_1$ und $R_2$ zusammen mit den dazwischenliegenden Kohlenstoff- und Stickstoffatomen einen heterocyclischen Ring darstellen; und

$R_3$ ein Wasserstoffatom oder eine Alkylgruppe bedeutet.

2. Verbindungen nach Anspruch 1, worin ein Substituent a para-ständig zur Etherverknüpfung angeordnet ist und die anderen Substituenten, soferne vorhanden, in ortho-Stellung zu der Etherbindung vorliegen.

3. Verbindungen nach Anspruch 1 oder 2, worin n den Wert 2 oder 3 aufweist; eine Gruppe A eine Halogenalkylgruppe ist, die zweite Gruppe A ein Halogenatom oder eine Nitro- oder Cyanogruppe bedeutet und die dritte Gruppe A, soferne vorhanden, ein Halogenatom ist.

4. Verbindungen nach Anspruch 3, worin die Halogenalkylgruppe eine Trifluormethylgruppe ist und das Halogenatom ein Chloratom ist.

5. Verbindungen nach einem der Ansprüche 1 bis 4, worin

X ein Wasserstoffatom darstellt,

Y eine Nitrogruppe bedeutet und

$R_3$ ein Wasserstoffatom oder eine Alkylgruppe mit bis zu 6 Kohlenstoffatomen bedeutet.

6. Verbindungen nach einem der vorstehenden Ansprüche, worin

$R_1$ ein Wasserstoffatom oder eine gegebenenfalls chlorierte Alkylgruppe mit bis zu 8 Kohlenstoffatomen bedeutet; und

Z ein Wasserstoffatom oder eine Methyl-, Cyano-, Carboxy-, Alkylamino- oder Phenylsulfonylgruppe; eine Alkoxycarbonyl- oder Aralkoxycarbonylgruppe; eine Alkanoylgruppe; oder eine N-substituierte Carbamoylgruppe der Formel $CONR_4R_5$, worin $R_4$ ein Wasserstoffatom oder eine Alkylgruppe mit bis zu 6 Kohlenstoffatomen bedeutet und $R_5$ eine Alkylgruppe mit bis zu 8 Kohlenstoffatomen bedeutet, die gegebenenfalls einen Alkoxy- oder Alkylamino-Substituenten trägt; eine Cyclopropylgruppe; oder eine Aminogruppe bedeutet, die gegebenenfalls einen Alkyl-, Alkenyl- oder Alkinylsubstituenten aufweist.

7. Verbindungen nach einem der vorstehenden Ansprüche, worin $R_2$ ein Wasserstoffatom, eine Alkylgruppe mit bis zu 8 Kohlenstoffatomen, die gegebenenfalls einen Halogen- oder Alkoxysubstituenten trägt; eine Cyclopropyl- oder Cyclohexylgruppe, die gegebenenfalls einen Alkoxycarbonylsubstituenten trägt; eine Alkenyl- oder Alkinylgruppe mit bis zu 8 Kohlenstoffatomen; eine Methyl- oder Ethylgruppe, die eine Alkoxycarbonyl-, Carbamoyl- oder Alkylaminogruppe trägt; eine Phenylgruppe; eine Alkoxygruppe; einen Thiophenring, der gegebenenfalls einen Alkoxycarbonylsubstituenten trägt; oder eine Aminogruppe, die gegebenenfalls durch eine Alkyl-, Phenyl-, Acetyl-, Benzoyl- oder Alkoxycarbonylgruppe substituiert ist, bedeutet.

8. Verbindungen nach einem der Ansprüche 1 bis 6, worin

$R_1$ und $R_2$, zusammen mit dem dazwischenliegenden Stickstoffatom, eine Azidogruppe oder einen stickstoffhaltigen heterocyclischen Ring, ausgewählt unter Pyrrolidin, Piperidin, Hydrooxazin oder Triazol bedeuten; oder

$R_1$, $R_2$ und Z, zusammen mit den dazwischenliegenden Stickstoff- und Kohlenstoffatomen, einen Diazol- oder Hydrodiazinring darstellen.

9. Verfahren zur Herstellung einer Verbindung der allgemeinen Formel I, wie in Anspruch 1 definiert,

welches ein Umsetzen eines Amins der Formel $HNR_1R_2$ mit einem Hydrazonylhalogenid der allgemeinen Formel II:

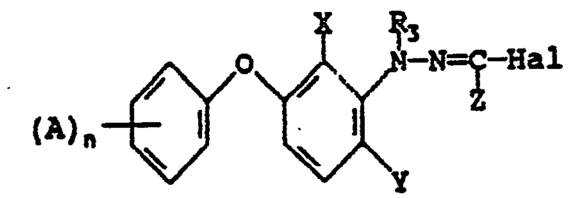

II

umfaßt, worin Hal ein Halogenatom bedeutet; und die anderen Substituenten die in Anspruch 1 definierten Bedeutungen besitzen.

10. Verfahren nach Anspruch 9, worin Hal ein Chloratom bedeutet und die Umsetzung unter Rückfluß ausgeführt wird.

11. Verfahren nach Anspruch 9 oder 10, worin das als Ausgangsmaterial eingesetzte Hydrazonylhalogenid der Formel II, worin Z eine andere Bedeutung als Alkyl oder Wasserstoff aufweist, durch Diazotieren eines Anilinderivats der allgemeinen Formel III:

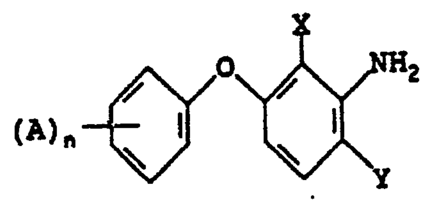

III ,

worin n, A, X und Y wie in Anspruch 1 definiert sind, und Kuppeln mit einer Halogenacylverbindung der allgemeinen Formel IV:

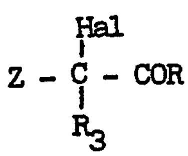

(IV),

worin
Hal ein Halogenatom bedeutet,
R eine Alkylgruppe darstellt und
Z und $R_3$ wie in Anspruch 1 definiert sind, hergestellt wird.

12. Verfahren nach Anspruch 11, worin die Halogenacylverbindung die Formel $Z-CR_3Cl-COCH_3$ aufweist.

13. Verfahren nach Anspruch 11 oder 12, worin Z in Formel IV eine Alkoxycarbonylgruppe bedeutet und das resultierende Hydrazonylhalogenid der Formel II mit überschüssigem Amin zu einer Verbindung der Formel I umgesetzt wird, worin Z eine Alkylcarbamoylgruppe darstellt.

14. Verfahren nach Anspruch 11 oder 12, worin Z in Formel IV eine Sulfonylgruppe darstellt und das resultierende Hydrazonylhalogenid der Formel II, worin Z Sulfonyl bedeutet, mit überschüssigem Amin zu einer Verbindung der Formel I umgesetzt wird, worin Z eine Alkylaminogruppe darstellt.

15. Verfahren nach einem der Ansprüche 11 bis 14, worin die Diazotierung bei einer Temperatur zwischen 0 und 10°C ausgeführt wird.

16. Verfahren nach Anspruch 9 oder 10, worin das als Ausgangsmaterial eingesetzte Hydrazonylhalogenid der Formel II, worin Z für Alkyl oder Wasserstoff steht, durch Umsetzen eines Hydrazins der Formel $R_3NH-NH_2$ mit einer Nitroverbindung der Formel V:

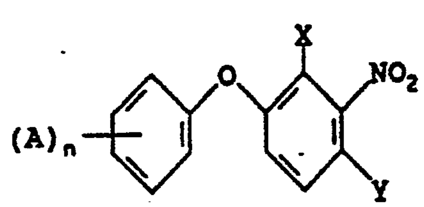

V,

Acylieren des gebildeten Phenylhydrazins durch Umsetzung mit einer Säure oder ihrem Anhydrid oder

einem Acylhalogenid der Formel ZCOOH oder ZCOHal, worin Z für Alkyl oder Wasserstoff steht und Hal ein Halogenatom bedeutet, und anschließende Reaktion mit Phosphorpentachlorid hergestellt wird.

17. Verfahren nach Anspruch 16, worin die Umsetzung der Nitro- und Hydrazinverbindungen und die anschließende Acylierung jeweils in einem inerten Lösungsmittel ausgeführt werden.

18. Verfahren nach Anspruch 16 oder 17, worin die Acylierung in Gegenwart eines Halogenwasserstoffakzeptors ausgeführt wird.

19. Herbizide Zusammensetzung, welche eine Verbindung, wie in einem der Ansprüche 1 bis 8 beansprucht, zusammen mit einem Träger umfaßt.

20. Zusammensetzung nach Anspruch 19, welche wenigstens zwei Träger umfaßt, von denen wenigstens einer ein oberflächenaktives Mittel ist.

21. Verfahren zur Bekämpfung von unerwünschtem Pflanzenwuchs an einem Ort, welches ein Behandeln des Ortes mit einer Verbindung, wie in einem der Ansprüche 1 bis 8 beansprucht, oder mit einer Zusammensetzung, wie in Anspruch 19 oder 20 beansprucht, umfaßt.

22. Die Verwendung einer Verbindung, wie in einem der Ansprüche 1 bis 8 beansprucht, als ein Herbizid.

**Patentansprüche für den Vertragsstaat: AT**

1. Herbizide Zusammensetzung, welche einen Hydrazinodiphenylether mit der allgemeinen Formel I:

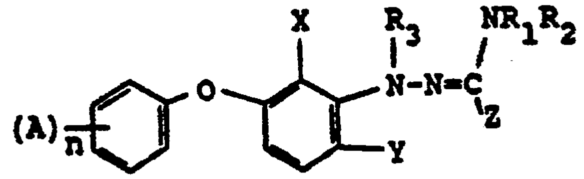

I

worin

n den Wert 0, 1, 2 oder 3 aufweist; jede Gruppe A, die gleich oder verschieden sein kann, wenn n größer als 1 ist, unabhängig voneinander ein Halogenatom oder eine Cyano-, Nitro-, Alkyl- oder Halogenalkylgruppe darstellt;

X ein Wasserstoffatom bedeutet, oder, wenn n den Wert 0 aufweist, in alternativer Weise ein Halogenatom bedeuten kann;

Y ein Halogenatom darstellt, oder eine Cyanogruppe oder Nitrogruppe bedeutet;

$R_1$ ein Wasserstoffatom, eine Alkyl-, Cycloalkyl-, Alkenyl- oder Alkinylgruppe, eine durch ein Halogenatom oder eine Cyano- oder Alkoxygruppe substituierte Alkylgruppe oder eine gegebenenfalls substituierte Arylgruppe darstellt;

$R_2$ ein Wasserstoffatom, eine Alkyl-, Alkoxy-, Cycloalkyl-, Alkenyl- oder Alkinylgruppe, eine durch ein Halogenatom oder eine Cyano-, Alkoxy-, Alkylamino-, Acylamino-, Alkoxycarbonyl- oder Acylgruppe substituierte Alkylgruppe, eine gegebenenfalls substituierte Aryl- oder heterocyclische Gruppe oder eine Aminogruppe, die gegebenenfalls durch eine Alkyl-, Aryl-, Alkanoyl-, Aroyl- oder Alkoxycarbonylgruppe substituiert ist, darstellt oder $R_1$ und $R_2$ zusammen mit dem dazwischenliegenden Stickstoffatom eine Azidogruppe oder einen heterocyclischen Ring bedeuten;

Z ein Wasserstoffatom oder eine Cyano-, Alkyl-, Alkylamino-, Carboxy-, Alkoxycarbonyl- oder Acylgruppe darstellt oder Z, $R_1$ und $R_2$ zusammen mit den dazwischenliegenden Kohlenstoff- und Stickstoffatomen einen heterocyclischen Ring darstellen; und

$R_3$ ein Wasserstoffatom oder eine Alkylgruppe bedeutet; zusammen mit einem Träger umfaßt.

2. Zusammensetzung nach Anspruch 1, worin in der allgemeinen Formel I ein Substituent A para-ständig zur Etherverknüpfung angeordnet ist und die anderen Substituenten, soferne vorhanden, in ortho-Stellung zu der Etherbindung vorliegen.

3. Zusammensetzung nach Anspruch 1 oder 2, worin in der allgemeinen Formel I n den Wert 2 oder 3 aufweist; eine Gruppe A eine Halogenalkylgruppe ist, die zweite Gruppe A ein Halogenatom oder eine Nitro- oder Cyanogruppe bedeutet und die dritte Gruppe A, soferne vorhanden, ein Halogenatom ist.

4. Zusammensetzung nach Anspruch 3, worin in der allgemeinen Formel I die Halogenalkylgruppe eine Trifluormethylgruppe ist und das Halogenatom ein Chloratom ist.

5. Zusammensetzung nach einem der Ansprüche 1 bis 4, worin in der allgemeinen Formel I

X ein Wasserstoffatom darstellt,

Y eine Nitrogruppe bedeutet und

$R_3$ ein Wasserstoffatom oder eine Alkylgruppe mit bis zu 6 Kohlenstoffatomen bedeutet.

6. Zusammensetzung nach einem der vorstehenden Ansprüche, worin in der allgemeinen Formel I

$R_1$ ein Wasserstoffatom oder eine gegebenenfalls chlorierte Alkylgruppe mit bis zu 8 Kohlenstoffatomen bedeutet; und

Z ein Wasserstoffatom oder eine Methyl-, Cyano-, Carboxy-, Alkylamino- oder Phenylsulfonylgruppe; eine Alkoxycarbonyl- oder Aralkoxycarbonylgruppe; eine Alkanoylgruppe; oder eine N-substituierte Carbamoylguppe der Formel $CONR_4R_5$, worin $R_4$ ein Wasserstoffatom oder eine Alkylgruppe mit bis zu 6 Kohlenstoffatomen bedeutet und $R_5$ eine Alkylgruppe mit bis zu 8 Kohlenstoffatomen bedeutet, die gegebenenfalls einen Alkoxy- oder Alkylamino-Substituenten trägt; eine Cyclopropylgruppe; oder eine Aminogruppe bedeutet, die gegebenenfalls einen Alkyl-, Alkenyl- oder Alkinylsubstituenten aufweist.

7. Zusammensetzung nach einem der vorstehenden Ansprüche, worin in der allgemeinen Formel I $R_2$ ein Wasserstoffatom, eine Alkylgruppe mit bis zu 8 Kohlenstoffatomen, die gegebenenfalls einen Halogen- oder Alkoxysubstituenten trägt; eine Cyclopropyl- oder Cyclohexylgruppe, die gegebenenfalls einen Alkoxycarbonylsubstituenten trägt; eine Alkenyl- oder Alkinylgruppe mit bis zu 8 Kohlenstoffatomen; eine Methyl- oder Ethylgruppe, die eine Alkoxycarbonyl-, Carbamoyl- oder Alkylaminogruppe trägt; eine Phenylgruppe; eine Alkoxygruppe; einen Thiophenring, der gegebenenfalls einen Alkoxycarbonylsubstituenten trägt; oder eine Aminogruppe, die gegebenenfalls durch eine Alkyl-, Phenyl-, Acetyl-, Benzoyl- oder Alkoxycarbonylgruppe substituiert ist, bedeutet.

8. Zusammensetzung nach einem der Ansprüche 1 bis 6, worin in der allgemeinen Formel I

$R_1$ und $R_2$, zusammen mit dem dazwischenliegenden Stickstoffatom, eine Azidogruppe oder einen stickstoffhaltigen heterocyclischen Ring, ausgewählt unter Pyrrolidin, Piperidin, Hydrooxazin oder Triazol bedeuten; oder

$R_1$, $R_2$ und Z, zusammen mit den dazwischenliegenden Stickstoff- und Kohlenstoffatomen, einen Diazol- oder Hydrodiazinring darstellen.

9. Zusammensetzung nach einem der Ansprüche 1 bis 8, welche wenigstens zwei Träger umfaßt, von denen wenigstens einer ein oberflächenaktives Mittel ist.

10. Verfahren zur Herstellung einer Verbindung der allgemeinen Formel I, wie in Anspruch 1 definiert, welches ein Umsetzen eines Amins der Formel $HNR_1R_2$ mit einem Hydrazonylhalogenid der allgemeinen Formel II:

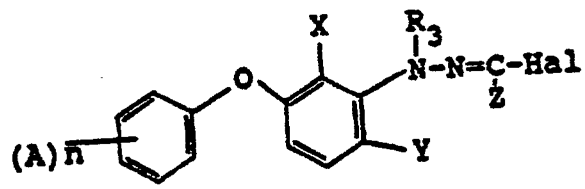

II

umfaßt, worin Hal ein Halogenatom bedeutet; und die anderen Substituenten die in Anspruch 1 definierten Bedeutungen besitzen.

11. Verfahren nach Anspruch 10, worin Hal ein Chloratom bedeutet und die Umsetzung unter Rückfluß ausgeführt wird.

12. Verfahren nach Anspruch 10 oder 11, worin das als Ausgangsmaterial eingesetzte Hydrazonylhalogenid der Formel II, worin Z eine andere Bedeutung als Alkyl oder Wasserstoff aufweist, durch Diazotieren eines Anilinderivats der allgemeinen Formel III:

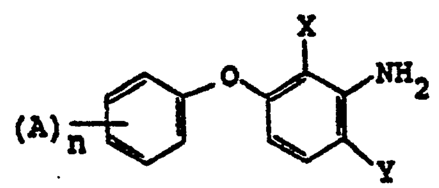

(III),

worin n, A, X und Y wie in Anspruch 1 definiert sind, und Kuppeln mit einer Halogenacylverbindung der allgemeinen Formel IV:

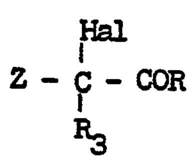

(IV),

worin

Hal ein Halogenatom bedeutet,

R eine Alkylgruppe darstellt und

Z und $R_3$ wie in Anspruch 1 definiert sind, hergestellt wird.

13. Verfahren nach Anspruch 12, worin die Halogenacylverbindung die Formel $Z{-}CR_3Cl{-}COCH_3$ aufweist.

14. Verfahren nach Anspruch 12 oder 13, worin Z in Formel IV eine Alkoxycarbonylgruppe bedeutet

und das resultierende Hydrazonylhalogenid der Formel II mit überschüssigem Amin zu einer Verbindung der Formel I umgesetzt wird, worin Z eine Alkylcarbamoylgruppe darstellt.

15. Verfahren nach Anspruch 12 oder 13, worin Z in Formel IV eine Sulfonylgruppe darstellt und das resultierende Hydrazonylhalogenid der Formel II, worin Z Sulfonyl bedeutet, mit überschüssigem Amin zu einer Verbindung der Formel I umgesetzt wird, worin Z eine Alkylaminogruppe darstellt.

16. Verfahren nach einem der Ansprüche 12 bis 15, worin die Diazotierung bei einer Temperatur zwischen 0 und 10°C ausgeführt wird.

17. Verfahren nach Anspruch 10 oder 11, worin das als Ausgangsmaterial eingesetzte Hydrazonylhalogenid der Formel II, worin Z für Alkyl oder Wasserstoff steht, durch Umsetzen eines Hydrazins der Formel $R_3NH{-}NH_2$ mit einer Nitroverbindung der Formel V:

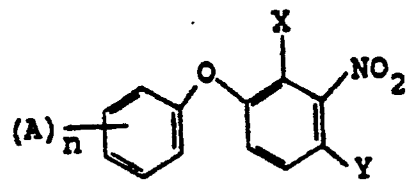

(V),

Acylieren des gebildeten Phenylhydrazins durch Umsetzung mit einer Säure oder ihrem Anhydrid oder einem Acylhalogenid der Formel ZCOOH oder ZCOHal, worin Z für Alkyl oder Wasserstoff steht und Hal ein Halogenatom bedeutet, und anschließende Reaktion mit Phosphorpentachlorid hergestellt wird.

18. Verfahren nach Anspruch 17, worin die Umsetzung der Nitro- und Hydrazinverbindungen und die anschließende Acylierung jeweils in einem inerten Lösungsmittel ausgeführt werden.

19. Verfahren nach Anspruch 17 oder 18, worin die Acylierung in Gegenwart eines Halogenwasserstoffakzeptors ausgeführt wird.

20. Verfahren zur Bekämpfung von unerwünschtem Pflanzenwuchs an einem Ort, welches ein Behandeln des Ortes mit einer Verbindung, wie in einem der Ansprüche 1 bis 8 beansprucht, oder mit einer Zusammensetzung, wie in einem der Ansprüche 1 bis 9 beansprucht, umfaßt.

21. Die Verwendung einer Verbindung, wie in einem der Ansprüche 1 bis 8 beansprucht, als ein Herbizid.

**Revendications pour les Etats contractants: BE, CH, DE, FR, GB, IT, LI, LU, NL, SE**

1. Ethers hydrazinodiphényliques de formule générale I:

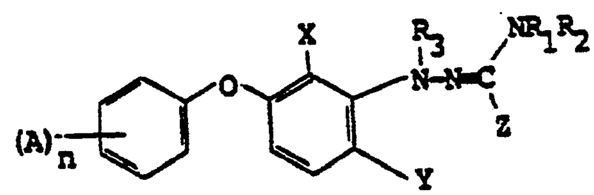

I

dans laquelle n est égal à 0, 1, 2 ou 3; chacun des groupes A, qui peuvent être identiques ou différents lorsque n est supérieur à 1, représente indépendamment un atome d'halogène ou un groupe cyano, nitro, alkyle ou halogénoalkyle;

X représente un atome d'hydrogène ou, lorsque n est nul, peut aussi représenter un atome d'halogène;

Y représente un atome d'halogène ou un groupe nitro ou cyano;

$R_1$ représente un atome d'hydrogène, un groupe alkyle, cycloalkyle, alcényle ou alcynyle, un groupe alkyle substitué par un atome d'halogène ou par un groupe cyano ou alcoxy, ou un groupe aryle éventuellement substitué;

$R_2$ représente un atome d'hydrogène, un groupe alkyle, alcoxy, cycloalkyle, alcényle ou alcynyle, un groupe alkyle substitué par un atome d'halogène ou un groupe cyano, alcoxy, alkylamino, acylamino, alcoxycarbonyle ou acyle, un groupe aryle ou hétérocyclique éventuellement substitué, ou un groupe amino éventuellement substitué par un groupe alkyle, aryle, alcanoyle, aroyle ou alcoxycarbonyle, ou bien $R_1$ et $R_2$ représentent ensemble, avec l'atome d'azote situé entre les deux, un groupe azido ou un noyau hétérocyclique;

Z représente un atome d'hydrogène ou un groupe cyano, alkyle, alkylamino, carboxy, alcoxycarbonyle ou acyle, ou bien Z, $R_1$ et $R_2$ représentent, avec les atomes de carbone et d'azote situés entre eux, un noyau hétérocyclique; et

$R_3$ représente un atome d'hydrogène ou un groupe alkyle.

2. Composés selon la revendication 1, dans lesquels l'un des substituants A est situé en para de la liaison éther et les autres substituants, lorsqu'ils sont présents, sont situés en ortho de la liaison éther.

3. Composés selon la revendication 1 ou 2, dans lesquels n est égal à 2 ou 3, l'un des groupes A est un groupe halogénoalkyle, le deuxième groupe A est un atome d'halogène ou un groupe nitro ou cyano, et le troisième groupe A, lorsqu'il est présent, est un atome d'halogène.

4. Composés selon la revendication 3, dans lesquels le groupe halogénoalkyle est un groupe trifluorométhyle et l'atome d'halogène est un atome de chlore.

5. Composés selon l'une quelconque des revendications 1-4, dans lesquels X représente un atome d'hydrogène, Y représente un groupe nitro et $R_3$ représente un atome d'hydrogène ou un groupe alkyle ayant jusqu'à 6 atomes de carbone.

6. Composés selon l'une quelconque des revendications précédentes, dans lesquels $R_1$ est un atome d'hydrogène ou un groupe alkyle éventuellement chloré ayant jusqu'à 8 atomes de carbone; et Z est un atome d'hydrogène ou un groupe méthyle, cyano, carboxyle, alkylamino ou phénylsulfonyle; un groupe alcoxycarbonyle ou aralcoxycarbonyle; un groupe alcanoyle; ou un groupe carbamoyle N-substitué de formule $CONR_4R_5$, dans laquelle $R_4$ représente un atome d'hydrogène ou un groupe alkyle ayant jusqu'à 6 atomes de carbone, et $R_5$ représente un groupe alkyle ayant jusqu'à 8 atomes de carbone, portant éventuellement un substituant alcoxy ou alkylamino; un groupe cyclopropyle; ou un groupe amino portant éventuellement un substituant alkyle, alcényle ou alcynyle.

7. Composés selon l'une quelconque des revendications précédentes, dans lesquels $R_2$ est un atome d'hydrogène, un groupe alkyle ayant jusqu'à 8 atomes de carbone, portant éventuellement un substituant halogéno ou alcoxy; un groupe cyclopropyle ou cyclohexyle, portant éventuellement un substituant alcoxycarbonyle; un groupe alcényle ou alcynyle ayant jusqu'à 8 atomes de carbone; un groupe méthyle ou éthyle portant un groupe alcoxycarbonyle, carbamoyle ou alkylamino; un groupe phényle; un groupe alcoxy; un noyau thiophène, portant éventuellement un substituant alcoxycarbonyle; ou un groupe amino éventuellement substitué par un groupe alkyle, phényle, acétyle, benzoyle ou alcoxycarbonyle.

8. Composés selon l'une quelconque des revendications 1-6, dans lesquels $R_1$ et $R_2$ représentent, avec l'atome d'azote situé entre les deux, un groupe azido ou un noyau hétérocyclique azoté choisi parmi la pyrrolidine, la pipéridine, l'hydro-oxazine ou le triazole; ou $R_1$, $R_2$ et Z représentent, avec les atomes d'azote et de carbone situés entre eux, un noyau diazole ou hydrodiazine.

9. Procédé de préparation d'un composé de formule générale I définie dans la revendication 1, qui comprend la réaction d'une amine de formule $HNR_1R_2$ avec un halogénure d'hydrazonyle de formule II:

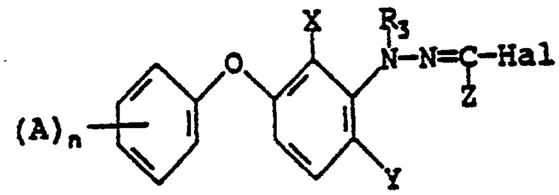

II

dans laquelle Hal représente un atome d'halogène; et les autres substituants ont les significations indiquées dans la revendication 1.

10. Procédé selon la revendication 9, dans lequel Hal représente un atome de chlore et la réaction est réalisée au reflux.

11. Procédé selon la revendication 9 ou 10, dans lequel l'halogénure d'hydrazonyle de départ de formule II, dans laquelle Z est autre qu'un groupe alkyle ou qu'un atome d'hydrogène, est préparé par diazotation d'un dérivé d'aniline de formule III:

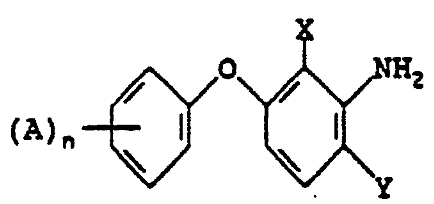

III

dans laquelle n, A, X et Y sont tels que définis dans la revendication 1, et par couplage avec un composé halogénoacyle de formule générale IV:

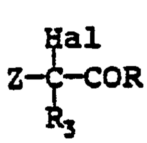

IV

dans laquelle Hal représente un atome d'halogène, R représente un groupe alkyle et $R_3$ est tel que défini dans la revendication 1.

12. Procédé selon la revendication 11, dans lequel le composé halogénoacyle répond à la formule $Z-CR_3Cl-COCH_3$.

13. Procédé selon la revendication 11 ou 12, dans lequel Z, dans la formule IV, représente un groupe alcoxycarbonyle et l'halogénure d'hydrazonyle résultant de formule II est mis à réagir avec un excès d'amine pour donner un composé de formule I dans laquelle Z représente un groupe alkylcarbamoyle.

14. Procédé selon la revendication 11 ou 12, dans lequel Z, dans la formule IV, représente un groupe sulfonyle et l'halogénure d'hydrazonyle résultant de formule II, dans laquelle Z est un groupe sulfonyle, est mis à réagir avec un excès d'amine pour donner un composé de formule I dans laquelle Z représente un groupe alkylamino.

15. Procédé selon l'une quelconque des revendications 11-14, dans lequel la diazotation est réalisée à une température comprise entre 0°C et 10°C.

16. Procédé selon la revendication 9 ou 10, dans lequel l'halogénure d'hydrazonyle de départ de formule II, dans laquelle Z est un groupe alkyle ou un atome d'hydrogène, est préparé par réaction d'une hydrazine de formule $R_3NH-NH_2$ avec un composé nitré de formule V:

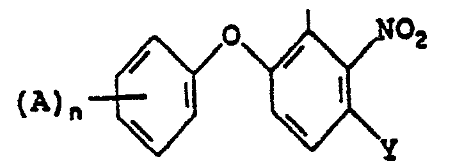

V

acylation de la phénylhydrazine résultante par réaction avec un acide, son anhydride ou un halogénure d'acyle, de formule ZCOOH ou ZCOHal, où Z est un hydrogène ou un groupe alkyle et Hal représente un atome d'halogène, suivie d'une réaction avec du pentachlorure de phosphore.

17. Procédé selon la revendication 16, dans lequel la réaction des composés nitré et hydrazine et l'acylation qui suit sont chacune réalisées dans un solvant inerte.

18. Procédé selon la revendication 16 ou 17, dans lequel l'acylation est réalisée en présence d'un accepteur d'halogénure d'hydrogène.

19. Composition herbicide, qui comprend un composé selon l'une quelconque des revendications 1 à 8, ainsi qu'un véhicule.

20. Composition selon la revendication 19, qui comprend au moins deux véhicules, dont l'un au moins est un agent tensio-actif.

21. Procédé de lutte contre la croissance indésirable de plantes en un lieu, qui comprend le traitement du lieu avec un composé selon l'une quelconque des revendications 1 à 8 ou avec une composition selon l'une quelconque des revendications 19 et 20.

22. Utilisation comme herbicide d'un composé selon l'une quelconque des revendications 1 à 8.

**Revendications pour l'Etat contractant: AT**

1. Composition herbicide, qui comprend un éther hydrazinodiphénylique de formule générale I:

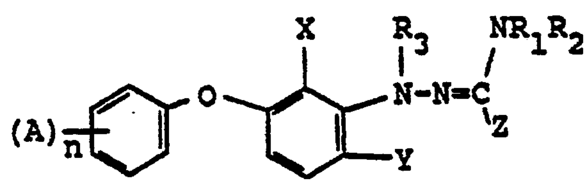

I

dans laquelle n est égal à 0, 1, 2 ou 3; chacun des groupes A, qui peuvent être identiques ou différents lorsque n est supérieur à 1, représente indépendamment un atome d'halogène ou un groupe cyano, nitro, alkyle ou halogénoalkyle;

X représente un atome d'hydrogène ou, lorsque n est nul, peut aussi représenter un atome d'halogène;

Y représente un atome d'halogène ou un groupe nitro ou cyano;

$R_1$ représente un atome d'hydrogène, un groupe alkyle, cycloalkyle, alcényle ou alcynyle, un groupe alkyle substitué par un atome d'halogène ou par un groupe cyano ou alcoxy, ou un groupe aryle éventuellement substitué;

$R_2$ représente un atome d'hydrogène, un groupe alkyle, alcoxy, cycloalkyle, alcényle ou alcynyle, un groupe alkyle substitué par un atome d'halogène ou un groupe cyano, alcoxy, alkylamino, acylamino, alcoxycarbonyle ou acyle, un groupe aryle ou hétérocyclique éventuellement substitué, ou un groupe amino éventuellement substitué par un groupe alkyle, aryle, alcanoyle, aroyle ou alcoxycarbonyle, ou bien $R_1$ et $R_2$ représentent ensemble, avec l'atome d'azote situé entre les deux, un groupe azido ou un noyau hétérocyclique;

Z représente un atome d'hydrogène ou un groupe cyano, alkyle, alkylamino, carboxy, alcoxycarbonyle ou acyle, ou bien Z, $R_1$ et $R_2$ représentent, avec les atomes de carbone et d'azote situés entre eux, un noyau hétérocyclique; et

$R_3$ représente un atome d'hydrogène ou un groupe alkyle; ainsi qu'un véhicule.

2. Composition selon la revendication 1, dans laquelle, dans la formule générale I, l'un des substituants A est situé en para de la liaison éther et les autres substituants, lorsqu'ils sont présents, sont situés en ortho de la liaison éther.

3. Composition selon la revendication 1 ou 2, dans laquelle, dans la formule générale I, n est égal à 2 ou 3, l'un des groupes A est un groupe halogénoalkyle, le deuxième groupe A est un atome d'halogène ou un groupe nitro ou cyano, et le troisième groupe A, lorsqu'il est présent, est un atome d'halogène.

4. Composition selon la revendication 3, dans laquelle, dans la formule générale I, le groupe halogénoalkyle est un groupe trifluorométhyle et l'atome d'halogène est un atome de chlore.

5. Composition selon l'une quelconque des revendications 1-4, dans laquelle, dans la formule générale I, X représente un atome d'hydrogène, Y représente un groupe nitro et $R_3$ représente un atome d'hydrogène ou un groupe alkyle ayant jusqu'à 6 atomes de carbone.

6. Composition selon l'une quelconque des revendications précédentes, dans laquelle, dans la formule générale I, $R_1$ est un atome d'hydrogène ou un groupe alkyle éventuellement chloré ayant jusqu'à 8 atomes de carbone; et Z est un atome d'hydrogène ou un groupe méthyle, cyano, carboxyle, alkylamino ou phénylsulfonyle; un groupe alcoxycarbonyle ou aralcoxycarbonyle; un groupe alcanoyle; ou un groupe carbamoyle N-substitué de formule $CONR_4R_5$, dans lequel $R_4$ représente un atome d'hydrogène ou un groupe alkyle ayant jusqu'à 6 atomes de carbone, et $R_5$ représente un groupe alkyle ayant jusqu'à 8 atomes de carbone, portant éventuellement un substituant alcoxy ou alkylamino; un groupe cyclopropyle; ou un groupe amino portant éventuellement un substituant alkyle, alcényle ou alcynyle.

7. Composition selon l'une quelconque des revendications précédentes, dans laquelle, dans la formule générale I, $R_2$ est un atome d'hydrogène, un groupe alkyle ayant jusqu'à 8 atomes de carbone, portant éventuellement un substituant halogéno ou alcoxy; un groupe cyclopropyle ou cyclohexyle, portant éventuellement un substituant alcoxycarbonyle; un groupe alcényle ou alcynyle ayant jusqu'à 8 atomes de carbone; un groupe méthyle ou éthyle portant un groupe alcoxycarbonyle, carbamoyle ou alkylamino; un groupe phényle; un groupe alcoxy; un noyau thiophène, portant éventuellement un substituant alcoxycarbonyle; ou un groupe amino éventuellement substitué par un groupe alkyle, phényle, acétyle, benzoyle ou alcoxycarbonyle.

8. Composition selon l'une quelconque des revendications 1-6, dans laquelle, dans la formule générale I, $R_1$ et $R_2$ représentent, avec l'atome d'azote situé entre les deux, un groupe azido ou un noyau hétérocyclique azoté choisi parmi la pyrrolidine, la pipéridine, l'hydro-oxazine ou le triazole; ou $R_1$, $R_2$ et Z représentent, avec les atomes d'azote et de carbone situés entre eux, un noyau diazole ou hydrodiazine.

9. Composition selon l'une quelconque des revendications 1-8, qui comprend au moins deux véhicules, dont l'un au moins est un agent tensio-actif.

10. Procédé de préparation d'un composé de formule générale I définie dans la revendication 1, qui comprend la réaction d'une amine de formule $HNR_1R_2$ avec un halogénure d'hydrazonyle de formule II:

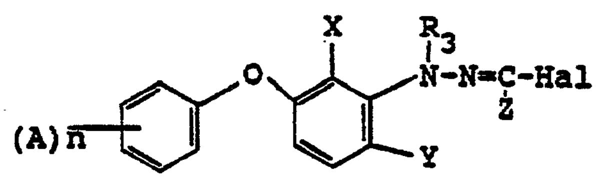 II

dans laquelle Hal représente un atome d'halogène; et les autres substituants ont les significations indiquées dans la revendication 1.

11. Procédé selon la revendication 10, dans lequel Hal représente un atome de chlore et la réaction est réalisée au reflux.

12. Procédé selon la revendication 10 ou 11, dans lequel l'halogénure d'hydrazonyle de départ de formule II, dans laquelle Z est autre qu'un groupe alkyle ou qu'un atome d'hydrogène, est préparé par diazotation d'un dérivé d'aniline de formule III:

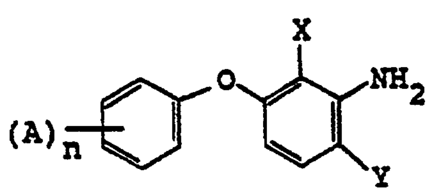 III

dans laquelle n, A, X et Y sont tels que définis dans la revendication 1, et par couplage avec un composé halogénoacyle de formule générale IV:

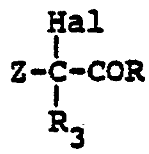

IV

dans laquelle Hal représente un atome d'halogène, R représente un groupe alkyle et $R_3$ est tel que défini dans la revendication 1.

13. Procédé selon la revendication 12, dans lequel le composé halogénoacyle répond à la formule $Z\text{-}CR_3Cl\text{-}COCH_3$.

14. Procédé selon la revendication 12 ou 13, dans lequel Z, dans la formule IV, représente un groupe alcoxycarbonyle et l'halogénure d'hydrazonyle résultant de formule II est mis à réagir avec un excès d'amine pour donner un composé de formule I dans laquelle Z représente un groupe alkylcarbamoyle.

15. Procédé selon la revendication 12 ou 13, dans lequel Z, dans la formule IV, représente un groupe sulfonyle et l'halogénure d'hydrazonyle résultant de formule II, dans laquelle Z est un groupe sulfonyle, est mis à réagir avec un excès d'amine pour donner un composé de formule I dans laquelle Z représente un groupe alkylamino.

16. Procédé selon l'une quelconque des revendications 12–15, dans lequel la diazotation est réalisée à une température comprise entre 0°C et 10°C.

17. Procédé selon la revendication 10 ou 11, dans lequel l'halogénure d'hydrazonyle de départ de formule II, dans laquelle Z est un groupe alkyle ou un atome d'hydrogène, est préparé par réaction d'une hydrazine de formule $R_3NH\text{-}NH_2$ avec un composé nitré de formule V:

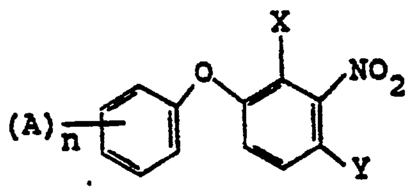

V

acylation de la phénylhydrazine résultante par réaction avec un acide, son anhydride ou un halogénure d'acyle, de formule ZCOOH ou ZCOHal, où Z est un hydrogène ou un groupe alkyle et Hal représente un atome d'halogène, suivie d'une réaction avec du pentachlorure de phosphore.

18. Procédé selon la revendication 17, dans lequel la réaction des composés nitré et hydrazine et l'acylation qui suit sont chacune réalisées dans un solvant inerte.

19. Procédé selon la revendication 17 ou 18, dans lequel l'acylation est réalisée en présence d'un accepteur d'halogénure d'hydrogène.

20. Procédé de lutte contre la croissance indésirable de plantes en un lieu, qui comprend le traitement du lieu avec une composition selon l'une quelconque des revendications 1 à 9.

21. Utilisation comme herbicide d'une composition selon l'une quelconque des revendications 1 à 8.